# FASCICULE DE BREVET EUROPEEN

(11) **EP 3 294 860 B1**
(45) Date de publication et mention de la délivrance du brevet: **08.01.2020**
(21) Numéro de dépôt: 16725058.8
(22) Date de dépôt: 12.05.2016
(51) Int. Cl.: C12M 1/107, C12M 1/00, C12M 1/26, A01C 3/04

(54) **DISPOSITIF DE MÉTHANISATION À PARTIR DE BIOMASSE SOLIDE ET PROCÉDÉ DE PRODUCTION DE BIOGAZ CORRESPONDANT**
VORRICHTUNG FÜR ANAEROBE GÄRUNG MIT FESTER BIOMASSE UND ENTSPRECHENDES VERFAHREN ZUR HERSTELLUNG VON BIOGAS
DEVICE FOR ANAEROBIC DIGESTION USING SOLID BIOMASS AND CORRESPONDING METHOD FOR PRODUCING BIOGAS

(30) Priorité: 12.05.2015 FR 1554268
(43) Date de publication de la demande: 21.03.2018
(73) Titulaire: YANNCO, 92800 Puteaux (FR)
(72) Inventeur: MERCIER, Yann, 92800 Puteaux (FR)
(74) Mandataire: Casalonga
(86) Numéro de dépôt international: PCT/EP2016/060627
(87) Numéro de publication internationale: WO 2016/180910

(56) Documents cités:
- EP-A1- 1 428 868
- EP-A2- 0 822 251
- WO-A2-2011/020000
- BE-A- 427 335

## Description

La présente invention concerne un digesteur de méthanisation destiné à la production de biogaz comprenant, au niveau du toit du digesteur, un espace d'introduction et d'évacuation de biomasse solide.

L'invention concerne également un procédé de production de biogaz ainsi qu'une installation de méthanisation mettant en œuvre une unité de digestion principale destinée notamment à alimenter en digestat liquide plusieurs digesteurs de méthanisation contenant de la biomasse solide.

Au sens de la présente invention, on entend par biomasse solide, des matières organiques d'origine végétales, animales, bactériologiques ou fongiques, contenant un taux de matière sèche supérieur à 12%, de préférence supérieur à 15%, qui permettent la production de biogaz.

La biomasse solide peut être fibreuse, notamment pailleuse, et/ou hétérogène, et/ou contenir des éléments indésirables, notamment des cailloux, du gravier, du sable, des barbelés, des ficelles, des pièces métalliques provenant d'outils agricoles ou encore des sacs plastiques, des éléments d'emballage et de conditionnement, ou encore des déchets urbains, éventuellement mal triés.

La méthanisation (encore appelée digestion anaérobie) est un procédé biologique naturel qui met en œuvre la dégradation des matières organiques par plusieurs types de micro-organismes, en particulier des bactéries, dans des conditions contrôlées et ceci en l'absence d'oxygène.

Un tel procédé conduit, d'une part, à la production de biogaz, qui correspond à un mélange gazeux composé majoritairement de méthane et de dioxyde de carbone, et, d'autre part, à la production d'un produit humide riche en matières organiques partiellement stabilisées appelé digestat, qui peut être ensuite utilisé comme amendement organique.

Le biogaz présente l'avantage d'être un gaz convertible en énergie renouvelable et peut par exemple servir dans la production d'électricité et/ou de chaleur ou dans la production de carburant. Le biogaz peut également être injecté dans un réseau de gaz naturel après épuration.

Le digestat peut subir un traitement de séparation de phase liquide/solide dont la fraction solide (appelé digestat solide) est plus riche en matières organiques et en éléments phosphatés et la fraction liquide (appelé digestat liquide) est plus riche en azote ammoniacal et en potassium. Les digestats liquides et solides peuvent être stockés, et/ou traités et/ou épandus séparément.

Ainsi le procédé biologique de méthanisation permet de valoriser des matières organiques, en produisant une énergie renouvelable, et de diminuer les émissions de gaz à effet de serre, en captant le méthane issu de la dégradation de ces matières, et en substituant l'emploi d'énergies fossiles et/ou d'engrais chimiques.

Les matières organiques, susceptibles de produire du biogaz par le biais d'une méthanisation, peuvent être des déchets provenant par exemple du secteur agricole et/ou agro-industriel et/ou être d'origine urbaine. En particulier, le fumier, provenant de l'exploitation agricole d'élevages, est une matière organique, issue des déjections animales mélangées à la paille des litières, qui constitue une source majeure et particulièrement intéressante dans la production de biogaz. A cet égard, la méthanisation dans le secteur agricole à partir de matières organiques, en particulier de pailles et/ou de fumier, représente une activité qui se développe de plus en plus à l'heure actuelle.

La méthanisation est couramment mise en œuvre à l'intérieur d'un réacteur appelé digesteur correspondant le plus souvent à une cuve, ayant une forme le plus souvent essentiellement cylindrique, mais parfois parallélépipédique, verticale ou horizontale, qui est destinée à contenir les matières organiques à traiter, en particulier du fumier et/ou des pailles et/ou des déchets d'origine industrielle, afin de produire du biogaz et du digestat.

Les différents procédés de méthanisation peuvent être répartis en deux grandes familles en fonction de la nature des déchets à dégrader, à savoir les procédés de méthanisation en voie liquide et les procédés de méthanisation en voie sèche.

En premier lieu, le procédé de méthanisation en voie liquide est généralement mis en œuvre pour traiter des déchets dont le mélange a un taux moyen de matière sèche inférieur à 15%, correspondant par exemple à des mélanges de boues liquides ou de graisses ou des mélanges principalement composés de lisier. Un tel procédé peut être aussi utilisé pour traiter des matières organiques solides qui ont été mélangées avec de l'eau ou du digestat liquide.

Le procédé de méthanisation en voie liquide consiste notamment à acheminer de manière généralement continue les matières organiques à traiter, le plus souvent au moyen de systèmes de pompage ou de vis sans fin à l'intérieur d'un digesteur qui est généralement hermétiquement fermé par l'intermédiaire d'un toit ou d'une membrane souple. Les matières organiques à traiter sont continuellement brassées à l'intérieur du digesteur, souvent par un ou plusieurs agitateurs, pouvant être à hélices ou à pâles, ou parfois par des injections de gaz, notamment de biogaz, afin d'éviter les phénomènes de décantation de la biomasse au fond du digesteur et/ou les phénomènes de flottation et de croûtage de la biomasse en surface du liquide dans le digesteur, et de favoriser la formation de biogaz et le contact avec les micro-organismes. Le digesteur peut éventuellement être chauffé dans des températures comprises entre 20 et 60°C, en particulier dans des températures comprises entre 35 et 55°C. Les matières organiques séjournent en moyenne plusieurs semaines dans le digesteur, typiquement pendant une période pouvant aller de 20 à 80 jours.

Le biogaz, produit au cours de cette réaction de méthanisation, est généralement stocké à pression atmosphérique dans une membrane souple étanche fixée au-dessus du digesteur. La membrane se présente alors sous la forme d'un dôme contenant un ciel gazeux au-dessus de la paroi verticale du digesteur. Plus rarement, le biogaz peut être stocké dans un gazomètre, souvent une poche de stockage souple située à côté du digesteur.

Ce procédé de méthanisation en voie liquide présente notamment l'inconvénient que les déchets à dégrader peuvent être difficiles à manipuler et à préparer pour leur acheminement vers le digesteur et nécessitent une dépense d'énergie importante pour être brassés de manière continue à l'intérieur du digesteur.

Dans les procédés de méthanisation en voie liquide qui incluent l'introduction de matières organiques solides, les matières organiques solides sont souvent broyées avant d'être incorporées dans la cuve de digestion afin de faciliter l'acheminement des matières organiques au sein du digesteur ainsi que leur brassage. Parfois, les matières organiques solides font l'objet d'un processus de tri préalable afin d'en retirer le plus possible d'objets indésirables.

Ce type de procédé de méthanisation en voie liquide continue présente souvent des difficultés au niveau de l'alimentation de la biomasse car il est souvent nécessaire de traiter préalablement les déchets avant leur introduction dans le digesteur, notamment en broyant la partie solide de la biomasse et en extrayant les matières indésirables. De plus, le système de brassage au sein du digesteur peut être endommagé du fait de la nature des matières organiques ou des matières indésirables.

En second lieu, le procédé de méthanisation, dit par voie sèche, est mis en œuvre pour traiter des déchets solides dont le mélange a un taux moyen de matière sèche généralement supérieur à 15%, notamment allant de 15% à 40%, correspondant par exemple à du fumier, de la paille, des déchets industriels ou urbains, ou des biomasses hétérogènes.

Un premier type de procédé de méthanisation par voie sèche consiste à acheminer en continu, notamment au moyen d'une trémie et d'un système de vis sans fin, ou plus rarement d'un système de pompage puissant, les matières organiques solides à traiter à l'intérieur d'un digesteur, le plus souvent disposé de manière horizontale. Les matières organiques solides à traiter sont brassées, le plus souvent lentement, à l'intérieur du digesteur qui peut être également chauffé dans des températures comprises entre 20 et 60°C, en particulier dans des températures comprises entre 35 et 55°C.

De plus, les matières organiques solides sont souvent broyées avant d'être incorporées dans la cuve de digestion afin de faciliter l'acheminement des matières organiques au sein du digesteur ainsi que leur brassage. Parfois les matières organiques solides font aussi l'objet d'un processus de tri préalable afin d'en retirer le plus possible d'objets indésirables.

Ce type de procédé de méthanisation en voie sèche continue présente souvent des difficultés au niveau de l'alimentation de la biomasse car il est souvent nécessaire de traiter préalablement les déchets avant leur introduction dans le digesteur, notamment en les broyant et en extrayant les matières indésirables. De plus, le système de brassage au sein du digesteur peut être endommagé du fait de la nature des matières organiques ou des matières indésirables.

Un second type de procédé de méthanisation en voie sèche est un procédé discontinu, ou en bâchées. Il consiste à acheminer, souvent au moyens d'engins mécaniques de chargement/déchargement, par exemple de type chargeur à godets, les matières organiques solides à l'intérieur de cellules de méthanisation qui sont fermées une fois remplies. Ces cellules sont généralement des sortes de garages équipés de portes que l'on referme une fois le chargement réalisé. Les matières organiques sont ensuite aspergés ou douchées avec du digestat liquide, chargé en bactéries, afin de réaliser une percolation conduisant à la production de biogaz. Une fois la réaction terminée, les portes de chaque cellule sont ouvertes afin de récupérer le digestat solide, généralement au moyen des mêmes engins mécaniques que pour le chargement. Un tel procédé met généralement en œuvre plusieurs cellules de méthanisation ou digesteurs, alimentées en bâchées l'une après l'autre. Dans ce procédé, les cellules sont généralement destinées à fonctionner majoritairement en même temps même si elles ont été alimentées à des moments différents. Le biogaz, produit au cours de la réaction de méthanisation, est le plus souvent stocké séparément dans un gazomètre, souvent une poche de stockage souple située à côté des digesteurs.

Ce type de procédé de méthanisation en voie sèche discontinue pose notamment le problème de la gestion du biogaz, susceptible d'être rejeté dans l'atmosphère pendant les périodes de remplissage et de vidange des cellules de méthanisation. De plus, la réaction de méthanisation n'est pas convenablement optimisée car la biomasse, introduite dans ces cellules, n'est pas mélangée et souvent des chemins préférentiels de circulation du digestat liquide dans la biomasse solide se forment, empêchant une bonne diffusion du digestat liquide au sein de la biomasse solide à traiter, et donc empêchant un contact intime entre les bactéries et la biomasse, réduisant sensiblement le taux de dégradation de la biomasse solide.

Ainsi les matières organiques à traiter soulèvent souvent des difficultés au cours des différents procédés de méthanisation car ils présentent l'inconvénient de comporter de nombreux éléments indésirables et/ou d'être difficiles à manipuler et/ou à broyer.

En effet, la paille ainsi que le fumier, ou encore les biomasses hétérogènes, peuvent comporter des cailloux, du gravier, du sable, des barbelés, des ficelles, des pièces métalliques provenant d'outils agricoles ou encore des sacs plastiques, des éléments d'emballage ou de conditionnement. De tels éléments sont souvent difficiles à séparer de la biomasse à traiter et peuvent donc endommager de manière importante les installations composant l'unité de méthanisation. A titre d'exemple, ces éléments indésirables peuvent endommager les pompes, les vis sans fin et les broyeurs conduisant ainsi au remplacement partiel ou total des pièces d'usure et/ou des pièces principales selon des fréquences élevées, engendrant des coûts de maintenance élevés.

De plus, la paille et les produits pailleux, tels que les fumiers, la menu-paille, les cannes de maïs, etc., sont délicats à manipuler car la paille est abrasive, ce qui peut abîmer, dégrader ou user les équipements employés au cours des différentes étapes des procédés de méthanisation (étapes de broyage, de triage, de pompage, de pressage et/ou de brassage).

De tels dégâts sont donc susceptibles d'entraîner une diminution dans la production de biogaz, voire un arrêt de celle-ci, et d'induire des manques à gagner et/ou une augmentation des coûts de maintenance et d'exploitation de l'installation.

En outre, une fois introduits, la paille et les produits pailleux sont souvent difficiles à brasser dans le digesteur, car ils ont tendance à flotter et/ou à décanter et/ou à s'emmêler, ce qui engendre une dépense d'énergie importante, notamment une consommation électrique élevée. Une telle dépense énergétique impacte négativement sur la rentabilité de l'installation.

Ainsi le fumier, la paille et les produits pailleux sont des matières organiques qui peuvent s'avérer difficiles à valoriser en vue d'une méthanisation.

Afin de remédier à certains de ces inconvénients, la demande de brevet FR 2 990 951 décrit notamment un procédé de préparation d'un substrat de méthanisation à partir de biomasse fibreuse solide, telle que le fumier et la paille, comprenant une étape de dilution de la biomasse dans un liquide, en particulier de l'eau ou du digestat liquide, une étape d'hydrolyse et une étape de séparation de la matière sèche en suspension du liquide de manière à préparer un substrat de méthanisation. En particulier, ce procédé comprend une étape de broyage de la biomasse avant ou après dilution, par exemple mise en œuvre à l'aide d'une pompe broyeuse. Ce procédé a pour de but de retirer les éléments indésirables de la biomasse fibreuse solide par décantation et, par conséquent, d'optimiser la préparation du substrat de méthanisation avant de réaliser son acheminement dans le digesteur.

Cependant, la biomasse fibreuse solide reste encore trop souvent difficile à manipuler au cours de ce procédé. En effet, la paille s'avère compliquée à compresser ce qui peut engendrer des problèmes de serrage et de blocage au niveau des pompes et des presses et/ou des moyens de séparation.

Un tel procédé présente également l'inconvénient d'engendrer des dépenses d'énergie importantes afin de maintenir correctement la paille en suspension dans le liquide au cours de l'étape de dilution. En effet, on observe que la paille a tendance à s'emmêler, à s'agglomérer, à flotter et à ne pas se mélanger aisément dans le liquide.

De plus, ce procédé ne résout pas convenablement le problème survenant lors du brassage de la biomasse dans le digesteur. En effet, la paille reste encore trop difficile à brasser dans le digesteur car cette dernière flotte en surface et s'emmêle, ce qui conduit à un phénomène d'accumulation et de croûtage qui empêche notamment la bonne circulation du biogaz au sein de la cuve, ce qui complique sa récupération, et qui peut aboutir à la prise en masse complète de la biomasse solide présente dans le digesteur et la paralysie de

EP0822251 divulgue (fig. 1) un digesteur de méthanisation, destiné à la production de biogaz. Le digesteur comporte une cuve (42) pour contenir la biomasse solide (9), et au niveau de sa partie supérieure : au moins une zone d'admission de la biomasse solide et d'évacuation de la biomasse solide digérée résiduelle (abstract et col. 1,1.43-44), sur laquelle un toit (48) est apte à s'ouvrir et à se refermer, et ayant au moins une zone d'évacuation du biogaz (51,52). Le digesteur de D1 comprend un bouchon (16) dans le toit.

Ainsi les différentes unités de méthanisation mises en œuvre à l'heure actuelle présentent un certain nombre d'inconvénients quand elles doivent traiter en totalité ou en partie des biomasses solides.

Au vu de ce qui précède, l'invention a notamment pour but de valoriser plus efficacement la biomasse solide tout en minimisant les dépenses d'énergie, en réduisant les dépenses de personnel d'exploitation, en rendant non nécessaire l'achat et l'installation de certains équipements de préparation et d'agitation de la biomasse solide, en diminuant les risques d'endommagement des installations composant l'unité de méthanisation et en diminuant les coûts de maintenance et de préparation de la biomasse solide.

La présente invention a donc notamment pour objet un digesteur de méthanisation, destiné à la production de biogaz, comportant une cuve, apte à contenir de la biomasse solide, comportant au niveau de sa partie supérieure : au moins une zone d'admission de la biomasse solide et d'évacuation de la biomasse solide digérée résiduelle, sur laquelle un toit est apte à s'ouvrir et à se refermer, et au moins une zone d'évacuation du biogaz. De préférence, ces zones sont séparées l'une de l'autre.

La zone d'admission, située en particulier sur au moins une partie de la surface supérieure de la cuve sur laquelle le toit s'ouvre et se referme, permet l'incorporation de la biomasse solide à l'intérieur de la cuve et l'évacuation de la biomasse solide une fois la digestion terminée, c'est-à-dire du digestat solide (encore appelé biomasse solide digérée résiduelle). Le toit du digesteur est ainsi amovible. La zone d'admission permettant d'incorporer et d'évacuer la biomasse solide est de préférence distincte de la zone d'évacuation du biogaz.

La zone d'admission permet donc d'introduire directement la biomasse solide dans le digesteur sans avoir à effectuer une opération de séparation des éléments indésirables ou une dilution préalable dans un liquide ou un broyage, ce qui permet de diminuer les investissements dans les équipements de préparation de la biomasse solide, de diminuer les dépenses énergétiques, de maintenance et de personnel et les risques d'endommagement par rapport à des unités de méthanisation classiques.

En particulier, le digesteur selon l'invention permet de s'affranchir d'un dispositif visant à séparer les éléments indésirables de la biomasse solide, d'un dispositif de broyage et d'un dispositif d'agitation de la biomasse dans le digesteur.

Après introduction de la biomasse solide, et fermeture du toit, la cuve du digesteur est remplie avec du digestat liquide, qui est acheminé par l'intermédiaire d'une ou plusieurs pompes d'alimentation, en provenance d'une unité de digestion apte à stocker du digestat liquide et du biogaz (appelée unité de digestion principale et qui peut être composée d'un ou plusieurs ouvrages). Après remplissage de la cuve, d'abord par de la biomasse solide, puis par du digestat liquide, la méthanisation se déroule par percolation du digestat liquide à travers la biomasse solide et diffusion du digestat liquide dans la biomasse solide, laquelle est totalement immergée (et éventuellement partiellement mise en suspension), dans des conditions anaérobies au sein de la cuve du digesteur.

La digestion de la biomasse solide par percolation du digestat liquide à travers la biomasse solide et diffusion du digestat liquide dans la biomasse solide permet de s'affranchir de la nécessité d'agiter le mélange de biomasse solide et de biomasse liquide dans la cuve du digesteur, évitant ainsi des investissements en moyens mécaniques d'agitation et des coûts d'exploitation.

La zone d'évacuation du biogaz, située au niveau de la partie supérieure du digesteur, permet alors de récupérer un mélange de biogaz et de digestat liquide qui va ensuite être acheminé vers l'unité de digestion principale apte à stocker du biogaz et du digestat liquide.

De préférence, la zone d'évacuation du biogaz est localisée au niveau de la surface supérieure de la cuve du digesteur.

Une fois la méthanisation terminée, une vidange aérobie du digestat liquide contenu dans la cuve du digesteur est réalisée, au cours de laquelle l'espace vacant laissé par cette vidange est rempli par de l'air, et l'accès à travers la zone d'admission permet alors de récupérer facilement la biomasse solide digérée résiduelle, une fois la digestion terminée, en minimisant l'énergie dépensée au cours de cette opération. Il s'agit donc d'un procédé de méthanisation discontinu, ou en bâchées.

En d'autres termes, la zone d'admission constitue un orifice d'entrée et de sortie de la biomasse solide situé au niveau de la surface supérieure de la cuve du digesteur selon l'invention.

Ainsi le digesteur selon l'invention est relié, via un circuit d'alimentation en digestat liquide et un circuit d'évacuation du biogaz et du digestat liquide, à une unité de digestion principale apte à contenir du biogaz et du digestat liquide. De cette façon, une circulation permanente, en aller et retour, du digestat liquide peut être mise en œuvre entre le digesteur et l'unité de digestion principale.

Le digesteur selon l'invention permet donc de valoriser plus efficacement la biomasse solide en réduisant les investissements en divers équipements, les dépenses énergétiques, de personnel et de maintenance des équipements, l'usure des matériels et les risques d'endommagement.

Selon une caractéristique de l'invention, le digesteur est surmonté d'un moyen de préhension apte à permettre l'alimentation en biomasse solide et l'évacuation de la biomasse solide digérée résiduelle.

En d'autres termes, le moyen de préhension est apte à charger la biomasse solide dans le digesteur et décharger la biomasse solide digérée résiduelle, une fois la digestion terminée, à travers la zone d'admission du digesteur.

Le moyen de préhension permet donc de saisir la biomasse solide et de l'incorporer dans le digesteur sans avoir à effectuer une étape de tri ou de séparation préalable des éléments indésirables susceptibles d'endommager les installations composant l'unité de méthanisation, et sans avoir à effectuer une étape de broyage ni de dilution de la biomasse solide.

Le moyen de préhension permet aussi de récupérer la biomasse solide digérée résiduelle une fois la digestion terminée, c'est-à-dire le digestat solide, et de l'évacuer du digesteur.

Ainsi le moyen de préhension permet d'alimenter et de décharger le digesteur par le haut à travers la zone d'admission (respectivement d'évacuation) située sur au moins une partie de la surface supérieure de la cuve sur laquelle se referme le toit.

Le moyen de préhension peut également servir à ouvrir le toit du digesteur avant d'alimenter le digesteur en biomasse solide ou de décharger le digestat solide.

Le moyen de préhension peut également servir à fermer le toit du digesteur après les opérations d'alimentation du digesteur en biomasse solide ou de déchargement du digestat solide.

Le moyen de préhension est préférentiellement un grappin apte à saisir la biomasse solide par le biais d'une pluralité de crochets ou une benne apte à saisir de la biomasse plus pâteuse par le biais de godets.

Selon une caractéristique du digesteur, la zone d'évacuation du biogaz comprend au moins un élément de séparation perforé supérieur, situé au niveau de la partie supérieure de la cuve, apte à séparer, d'une part, le mélange composé de biogaz et de digestat liquide et d'autre part la biomasse solide.

La zone d'évacuation de biogaz permet donc également d'évacuer du digestat liquide par débordement.

En particulier, l'évacuation du biogaz peut se faire et se fait de préférence concomitamment à la recirculation du digestat liquide vers l'unité de digestion principale.

Avantageusement, l'élément de séparation perforé supérieur, apte à extraire le mélange à base de biogaz et de digestat liquide, est un tuyau perforé dont l'extrémité est obturée partiellement ou totalement.

Selon une autre caractéristique de l'invention, le digesteur comprend au moins un élément de séparation perforé inférieur, situé au niveau de la partie inférieure du digesteur, afin de séparer le digestat liquide et la biomasse solide notamment lors des vidanges aérobies et anaérobies du digestat liquide vers l'unité de digestion principale.

De préférence, les éléments de séparation perforés inférieur, situés au niveau de la partie inférieure de la cuve du digesteur, se trouvent en amont du système de vidange et d'évacuation du digestat liquide vers l'unité de digestion principale.

De préférence, l'élément de séparation perforé inférieur, situé au niveau de la partie inférieure du digesteur, est un plancher perforé pouvant être situé sur la totalité ou une partie du sol du digesteur et/ou est une virole perforée située en surépaisseur sur la virole ou le mur vertical de la cuve du digesteur.

En particulier, le plancher perforé peut correspondre à une couronne perforée occupant partiellement le sol du digesteur ce qui permet de minimiser les risques d'endommagement du plancher par le moyen de préhension, notamment lors de l'étape d'évacuation du digestat solide.

De préférence, l'élément de séparation perforé inférieur est une couronne perforée.

Selon une caractéristique de l'invention, le digesteur comprend une ouverture munie d'une vanne apte à obturer ou permettre le passage de l'air entre l'intérieur et l'extérieur de la cuve du digesteur et une vanne destinée à obturer ou permettre la circulation du biogaz et du digestat liquide entre le digesteur et l'unité de digestion principale.

La vanne, apte à obturer ou permettre le passage de l'air entre l'intérieur et l'extérieur de la cuve du digesteur, permet d'introduire de l'air à l'intérieur du digesteur, notamment au moment de la mise en œuvre d'une étape de vidange aérobie, et permet d'évacuer l'air au moment du remplissage du digesteur avec du digestat liquide après le chargement du digesteur en biomasse solide.

La vanne, apte à obturer ou permettre la circulation du biogaz et du digestat liquide, permet de les faire circuler dans les circuits d'acheminement reliés à l'unité de digestion principale.

L'unité de digestion principale peut correspondre à un digesteur apte à stocker conjointement du digestat liquide et du biogaz ou à deux cuves distinctes capables de stocker séparément du digestat liquide et du biogaz. Dans certains cas, l'unité de digestion principale peut comporter plusieurs digesteurs aptes à stocker conjointement du digestat liquide et du biogaz ou plusieurs cuves distinctes capables de stocker séparément du digestat liquide et du biogaz.

L'unité de digestion principale est distincte du digesteur selon l'invention.

Selon une caractéristique de l'invention, le toit apte à s'ouvrir et se fermer situé sur la partie supérieure du digesteur peut être surmonté d'un bouchon permettant d'assurer une meilleure étanchéité du toit et d'éviter les fuites éventuelles de biogaz. Dans certains cas, ce bouchon peut être un bouchon liquide, le liquide pouvant notamment être de l'eau ou du digestat liquide.

L'invention a également pour objet un procédé de production de biogaz comprenant successivement :
- une étape d'alimentation en biomasse solide, mise en œuvre par au moins un moyen de préhension, à travers une zone d'admission et d'évacuation située au niveau de la partie supérieure d'une cuve d'un digesteur et sur laquelle un toit est apte à s'ouvrir et à se refermer,
- une étape de remplissage de la cuve du digesteur avec un digestat liquide pour immerger la biomasse solide et éventuellement mettre en suspension une partie de ladite biomasse solide,
- une étape de digestion constituée d'une percolation du digestat liquide à travers la biomasse solide, et d'une diffusion du digestat liquide dans la biomasse solide, afin de mettre en contact intime les bactéries contenues dans le digestat liquide et la biomasse solide, dans des conditions anaérobies, pour générer puis récupérer le biogaz à travers une zone d'évacuation située dans la partie supérieure du digesteur,
- une étape de vidange aérobie du digestat liquide de la cuve du digesteur, durant laquelle le digestat liquide contenu dans le digesteur est vidangé. De préférence, cette vidange consiste en un pompage à partir du bas de la cuve du digesteur vers l'unité de digestion principale à travers l'élément de séparation inférieur. Durant cette étape, l'espace vacant laissé par l'évacuation du digestat liquide est rempli par de l'air en provenance de l'extérieur de la cuve. A l'issue de cette étape, la biomasse solide partiellement ou totalement digérée peut venir reposer sur le fond de la cuve du digesteur et/ou sur l'élément de séparation inférieur, et
- une étape d'évacuation de la biomasse solide digérée résiduelle après digestion, autrement dit du digestat solide, mise en œuvre par au moins un moyen de préhension, à travers la zone d'admission de la biomasse solide et d'évacuation de la biomasse solide digérée résiduelle du digesteur.

En d'autres termes, la cuve du digesteur est alimentée par le haut en biomasse solide à travers une zone sur laquelle le toit du digesteur s'ouvre et se referme.

Le procédé de production du biogaz présente donc l'avantage de ne pas nécessiter une étape de préparation et/ou de séparation des éléments indésirables et/ou de broyage et/ou de dilution préalable de la biomasse solide ni de nécessiter de brassage de la biomasse solide dans le digesteur, ce qui permet de minimiser les investissements en équipements et les dépenses énergétiques associées à de telles étapes ainsi que les risques éventuels d'endommagement des équipements du digesteur ou des équipements mis en œuvre au cours du procédé et de diminuer les coûts de personnel et de maintenance liés aux différentes étapes évitées.

Selon une caractéristique, entre l'étape de digestion de la biomasse solide et l'étape de vidange aérobie du digestat liquide, le procédé comprend au moins les étapes suivantes :
- une étape de vidange anaérobie du digestat liquide de la cuve du digesteur, durant laquelle le digestat liquide, contenu dans le digesteur, est vidangé. De préférence, cette vidange consiste en un pompage à partir du bas de la cuve du digesteur vers l'unité de digestion principale à travers l'élément de séparation inférieur. Durant cette étape, l'espace vacant laissé par l'évacuation du digestat liquide est rempli par du biogaz en provenance de l'unité de digestion principale. A l'issue de cette étape la biomasse solide partiellement ou totalement digérée vient reposer sur le fond de la cuve du digesteur, et
- une étape de re-remplissage de la cuve du digesteur avec du digestat liquide. De préférence, le digestat liquide provient de l'unité de digestion principale après vidange anaérobie.

Selon une caractéristique, entre l'étape de re-remplissage de la cuve du digesteur avec un digestat liquide et l'étape de vidange aérobie de la biomasse liquide, le procédé comprend en outre au moins une étape de digestion constituée par une percolation du digestat liquide dans la biomasse solide et une diffusion du digestat liquide dans la biomasse solide pour générer puis récupérer le biogaz à travers la zone d'évacuation du biogaz située dans la partie supérieure du digesteur.

En particulier, le procédé comporte une étape d'ouverture du toit du digesteur avant l'étape d'alimentation afin de permettre le remplissage par le haut de la cuve du digesteur avec de la biomasse solide.

La zone d'admission est ainsi située sur au moins une partie de la surface supérieure de la cuve sur laquelle le toit se referme.

Selon un mode de réalisation, le procédé comporte une étape de fermeture du toit sur la zone d'admission du digesteur préalablement à l'étape de remplissage de la cuve avec du digestat liquide.

Optionnellement, le moyen de préhension peut également ouvrir et éventuellement fermer le toit du digesteur.

Le digestat liquide est ensuite introduit à l'intérieur de la cuve par le biais d'une ou plusieurs pompes d'alimentation en provenance de l'unité de digestion principale apte à stocker du biogaz et du digestat liquide.

Selon un mode de réalisation, après l'étape de remplissage de la cuve par la biomasse solide à traiter et du digestat liquide, la vanne, apte à obturer ou permettre le passage de l'air entre l'intérieur et l'extérieur de la cuve du digesteur, est fermée de manière à ce que la réaction de méthanisation se déroule dans des conditions anaérobies.

L'étape de percolation conduit, au niveau de la partie supérieure du digesteur, à la formation d'un flux de biogaz et de digestat liquide.

La percolation est assurée par la mise en œuvre d'une ou plusieurs pompes de circulation située(s) entre l'unité de digestion principale et la cuve du digesteur.

En particulier, la ou les pompe(s) de circulation permet(tent) d'assurer une pression du digestat liquide sur la biomasse solide l'obligeant à la traverser ce qui favorise un contact intime entre les bactéries contenues notamment dans le digestat liquide et la biomasse solide à dégrader.

La percolation s'accompagne d'une diffusion du digestat liquide dans la biomasse solide, laquelle est favorisée par la pression exercée par le pompage du digestat liquide sur la biomasse solide. Cette diffusion favorise le contact intime entre les bactéries et la biomasse solide à traiter.

De plus, le pompage et la circulation forcée du digestat liquide entre l'unité de digestion principale et le digesteur présentent l'avantage de faire circuler une très grande quantité de digestat liquide à travers un volume plus faible de biomasse solide à traiter ce qui limite les risques d'acidose dans le digesteur, aux alentours de la biomasse solide à traiter.

En effet, dans les autres procédés de méthanisation, des quantités trop importantes de biomasse à traiter dans le digesteur peuvent conduire à une réaction d'acidogénèse excessive, pouvant provoquer une acidification du digestat liquide, ou acidose, laquelle entraine une inhibition partielle, voire totale, de la méthanogénèse et donc de la production de méthane. En synthèse, une telle réaction est provoquée lorsque la production d'acides gras volatils, induite par des bactéries acidogènes, est plus importante que la capacité de transformation de ces acides gras en méthane par les bactéries méthanogènes.

Selon un mode de réalisation, le procédé comprend, au moment de l'évacuation du biogaz vers l'unité de digestion principale et de la recirculation du digestat liquide vers l'unité de digestion principale, une étape de séparation, mise en œuvre au moyen d'au moins un élément de séparation perforé supérieur, de préférence un tuyau perforé ou un plafond perforé, permettant à la fois d'extraire le mélange composé de biogaz et de digestat liquide vers l'unité de digestion principale et de retenir dans le digesteur la biomasse fibreuse solide qui se trouve en suspension dans la cuve.

En particulier, pendant la phase de digestion anaérobie, la vanne permettant d'obturer ou de permettre la circulation du biogaz et du digestat liquide est ouverte afin de faire circuler le biogaz et le digestat vers l'unité de digestion principale. La vanne est notamment située en aval de l'élément de séparation supérieur et en amont ou sur les circuits d'acheminement du biogaz et du digestat liquide.

Selon un mode de réalisation, l'étape de vidange anaérobie du digestat liquide est mise en œuvre en vidant par le bas la cuve du digestat liquide ce qui provoque le tassement de la biomasse solide au fond de la cuve et permet d'éliminer les chemins préférentiels de percolation ainsi que les poches de biogaz bloquées dans la biomasse.

L'étape de vidange anaérobie peut être mise en œuvre en aspirant le digestat liquide par le biais de la ou les pompe(s) située(s) entre la cuve du digesteur et l'unité de digestion principale.

L'aspiration du digestat liquide peut de préférence avoir lieu en aval de l'élément perforé inférieur pour éviter que la biomasse solide soit entrainée avec le digestat liquide vers l'unité de digestion principale.

L'étape de vidange anaérobie permet au biogaz en provenance de l'unité de digestion principale de remplir l'ensemble du volume libéré dans la cuve en remplacement du digestat liquide qui est aspiré par la pompe vers l'unité de digestion principale.

Après l'étape de vidange anaérobie, une étape de re-remplissage de la cuve avec du digestat liquide est à nouveau mise en œuvre afin de poursuivre la réaction de méthanisation.

De la même façon que précédemment, l'étape de digestion conduit à la formation de biogaz, récupéré par le biais de l'élément de séparation perforé supérieur situé au niveau de la partie supérieure du digesteur.

Selon un mode de réalisation, l'étape de vidange aérobie du digestat liquide de la cuve du digesteur est réalisée à la fin du cycle de méthanisation.

Une telle étape est mise en œuvre en fermant la vanne permettant d'obturer ou de faire circuler le biogaz et le digestat liquide, et en ouvrant la vanne permettant d'obturer ou d'introduire l'air dans le digesteur et en vidant la cuve du digestat liquide depuis le bas de la cuve du digesteur vers l'unité de digestion principale.

L'étape de vidange aérobie est mise en œuvre en aspirant le digestat liquide par le biais de la ou les pompe(s) située(s) entre la cuve du digesteur et l'unité de digestion principale.

L'aspiration du digestat liquide peut avoir lieu de préférence en aval de l'élément perforé pour éviter que la biomasse solide soit entrainée avec le digestat liquide vers l'unité de digestion principale.

L'étape de vidange aérobie permet à l'air en provenance de l'extérieur de remplir l'ensemble du volume libéré dans la cuve en remplacement du digestat liquide qui est aspiré par la pompe vers l'unité de digestion principale.

Selon un mode de réalisation, le procédé comporte une étape d'ouverture du toit sur la zone d'admission de la biomasse solide préalablement à l'étape d'évacuation du digestat solide, correspondant à la biomasse solide digérée résiduelle après méthanisation.

L'étape d'évacuation de la biomasse solide digérée résiduelle peut être effectuée par le biais du moyen de préhension apte à saisir la biomasse solide digérée résiduelle après méthanisation.

Une telle étape présente l'avantage de pouvoir se dérouler en toute sécurité après la vidange aérobie du digestat liquide, en absence de contact avec le biogaz.

L'invention concerne encore un système de production de biogaz, comportant au moins un digesteur tel que défini précédemment, qui est ou sont relié(s) par le biais d'un circuit d'alimentation en digestat liquide et d'un circuit d'évacuation de biogaz et de digestat liquide, à au moins une unité de digestion principale apte à contenir du biogaz et du digestat liquide.

En d'autres termes, le système de production de biogaz correspond à une installation de méthanisation mettant en œuvre une unité de digestion principale destinée à alimenter en digestat liquide un ou plusieurs digesteurs de méthanisation, tel(s) que défini(s) précédemment, et à récupérer le biogaz produit par ce ou ces différents digesteurs.

Alternativement, la ou les unités de digestion principale peuvent être constituées chacune par deux ouvrages distincts, l'un destiné à contenir principalement du digestat liquide, l'autre destiné à contenir principalement du biogaz.

L'unité de digestion principale est différente du digesteur selon l'invention.

De préférence, le circuit d'évacuation de biogaz et de recirculation du digestat liquide vers l'unité de digestion principale comprend un circuit apte à acheminer du biogaz et un circuit apte à acheminer du digestat liquide.

De préférence, le circuit d'évacuation comprend un pot de séparation apte à séparer le biogaz et le digestat liquide.

L'unité de digestion principale ne contient pas de biomasse solide ou dans des quantités très faibles.

L'unité de digestion principale peut avoir une fonction complémentaire de production de biogaz. En effet, durant l'étape de digestion, la recirculation du digestat liquide en provenance du ou des digesteurs vers l'unité de digestion principale, après percolation du digestat liquide dans la biomasse solide, peut provoquer des entrainements avec le digestat liquide de matières fines solides non encore digérées et/ou des entrainements d'acide gras volatiles qui n'ont pas eu le temps d'être transformés en biogaz dans le digesteur. Dans ces conditions, la méthanisation résiduelle de ces matières se produit dans l'unité de digestion principale.

Comme indiqué précédemment, l'unité de digestion principale peut être composée d'un ou plusieurs ouvrages.

D'autres avantages et caractéristiques de l'invention apparaîtront à l'examen de la description détaillée d'un mode de réalisation de l'invention nullement limitatif, et des dessins annexés, sur lesquels :
- la figure 1 représente schématiquement une vue en coupe du digesteur, selon l'invention, relié à une unité de digestion principale stockant conjointement du biogaz et du digestat liquide,
- les figures 2 à 9 représentent schématiquement des vues en coupe du digesteur au cours des différentes étapes du procédé de production du biogaz,
- la figure 10 représente schématiquement une vue de dessus d'un digesteur selon l'invention,
- la figure 11 représente schématiquement une vue du digesteur, selon l'invention, relié à une unité de digestion principale composée de deux ouvrages,
- la figure 12 illustre schématiquement une vue de dessus d'un système de production de biogaz et de digestat comprenant une unité de digestion principale apte à alimenter en digestat liquide une pluralité de digesteurs, conformes à la présente invention, et à stocker le biogaz en provenance desdits digesteurs.

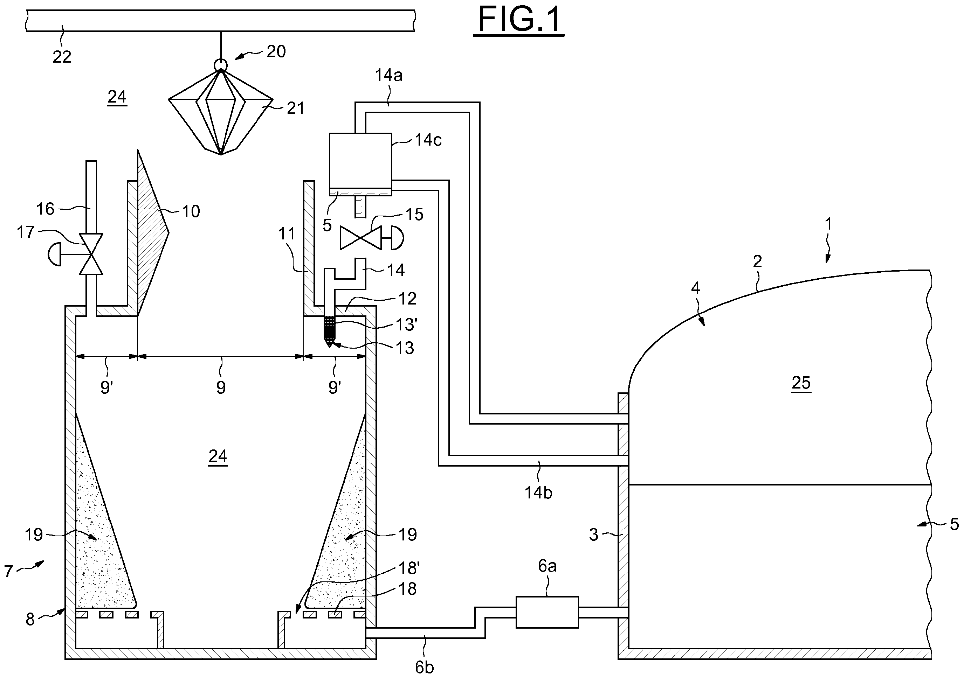

Sur la figure 1 est représentée de manière schématique, une unité de digestion principale 1, composée d'un seul ouvrage, et un digesteur 7 selon l'invention.

L'unité de digestion principale 1 comprend une cuve 3, ayant une forme essentiellement cylindrique, remplie de digestat liquide 5, qui est surmontée d'une membrane étanche 2 constituant un espace de stockage de gaz 4, lequel est rempli de biogaz 25. La membrane 2 occupe donc la partie en hauteur de l'unité de digestion principale 1 formant ainsi un dôme contenant un ciel gazeux. La cuve 3 correspond à une réserve de digestat liquide 5.

En variante, la cuve 3 peut présenter une forme cubique ou parallélépipédique.

La cuve 3 peut être fabriquée à partir d'acier, de béton ou de tout autre matériau tandis que la membrane étanche 2 peut être réalisée en polymère, notamment en polyéthylène, polypropylène ou chlorure de polyvinyle.

En variante, la membrane étanche 2 peut être surmontée par au moins une membrane supplémentaire de manière à ce que de l'air soit pris en sandwich entre la membrane supplémentaire et la membrane étanche 2.

L'unité de digestion principale 1 est reliée à un digesteur 7, selon l'invention, par l'intermédiaire d'une tuyauterie 6b sur laquelle est montée une pompe 6a. Le digesteur 7 comprend une cuve 8, de forme essentiellement cylindrique, dotée d'un orifice d'entrée 9, ayant une surface inférieure ou égale à la surface totale de la partie supérieure de la cuve 8, sur lequel un toit amovible 10 est apte à se refermer et/ou à s'ouvrir. En variante, la cuve 8 peut présenter une forme essentiellement cubique ou parallélépipédique.

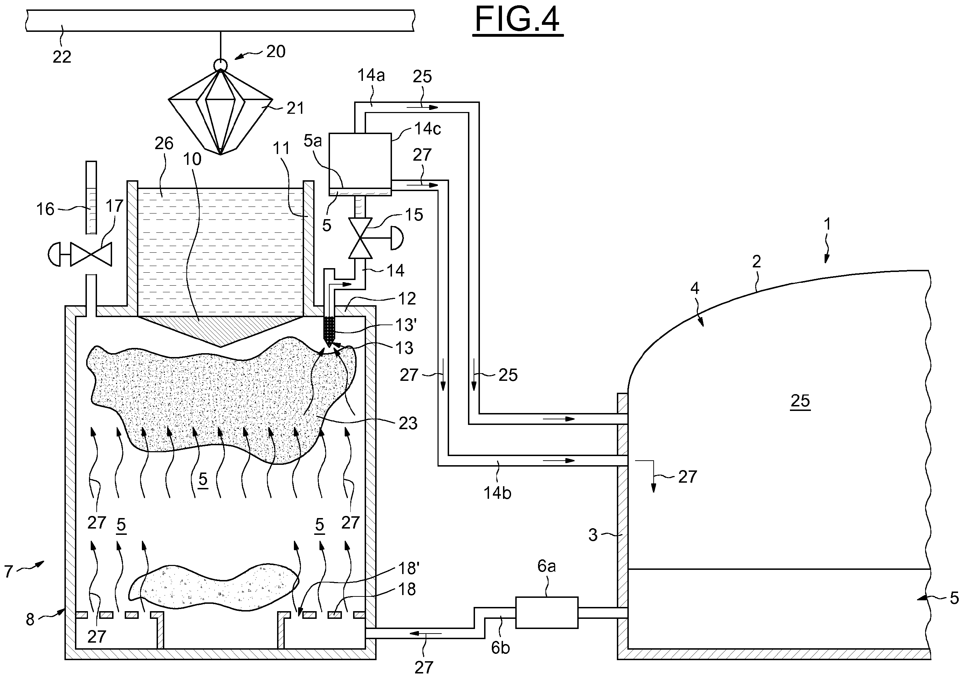

Au niveau de la périphérie de l'orifice d'entrée 9, la cuve 8 comporte une couronne 11 qui est en saillie vers l'extérieur par rapport à la surface supérieure 12 de la cuve 8. La couronne en saillie 11 peut représenter une position en butée permettant de maintenir le toit amovible 10 lorsqu'il est en position relevée. La couronne en saillie 11 peut également former un rempart de sécurité ou un muret de séparation permettant de protéger les éléments situés à l'extérieur de la couronne 11, notamment au cours de l'étape d'alimentation et d'évacuation de la biomasse. La couronne 11 peut représenter une enceinte dans laquelle sera situé un bouchon assurant l'étanchéité du toit (représenté sur la figure 4). Un tel bouchon peut être liquide ou solide. Dans le mode de réalisation illustré dans la figure 1, le toit amovible 10 est ouvert permettant à l'air de circuler à travers l'orifice d'entrée 9 et à l'intérieur de la cuve 8.

La pompe 6a permet non seulement d'alimenter le digesteur 7 en digestat liquide 5 provenant de l'unité de digestion principale 1 mais également de vidanger le digestat liquide 5 se trouvant à l'intérieur de la cuve 8 vers le digesteur principal 1. Dans le mode de réalisation illustré dans la figure 1, la tuyauterie 6b relie la cuve 3, remplie en digestat liquide 5, de l'unité de digestion principale 1, à la cuve 8 du digesteur 7, qui est vide.

Ainsi la tuyauterie 6b correspond à une tuyauterie d'alimentation, lors de l'étape de remplissage de la cuve 8 en digestat liquide 5, et à une tuyauterie de vidange de la cuve 8 lors des étapes vidanges anaérobies et aérobies.

La cuve 8 comporte, au niveau de sa partie supérieure, un élément de séparation perforé supérieur 13, de préférence un tuyau perforé, en saillie s'étendant de l'intérieur jusqu'au bord de la cuve 8. L'élément de séparation perforé supérieur 13 est positionné à l'extérieur de la couronne 11.

L'élément de séparation perforé supérieur 13 comprend une pluralité d'interstices 13'.

L'élément de séparation perforé supérieur 13 est connecté à une conduite 14 sur laquelle est montée une vanne 15 située en amont d'un pot de séparation 14c, lequel est relié à deux circuits distincts 14a et 14b qui sont reliés à leur extrémité à l'unité de digestion principale 1. La conduite 14 permet de récupérer un fluide issu de l'élément de séparation perforé supérieur 13 et la vanne 15 permet d'obturer ou de permettre la circulation du fluide dans le circuit d'acheminement 14. La vanne 15 se trouve en position fermée sur la figure 1.

Le circuit 14a, destiné à acheminer principalement le biogaz, se trouve au-dessus du circuit 14b, destiné à acheminer principalement le digestat liquide, ce qui facilite le transfert des différents fluides.

Le pot 14c, monté sur le circuit d'acheminement 14, permet de séparer plus efficacement le biogaz du digestat liquide lorsqu'ils sont extraits de la cuve 8 à travers l'élément de séparation 13.

Dans la figure 1, le pot 14c contient du digestat liquide 5 issu d'un précédent cycle de méthanisation.

Le circuit 14b peut comprendre une pompe de circulation (non représenté sur la figure 1) afin de faciliter l'acheminement du digestat liquide.

L'élément de séparation perforé supérieur 13 se trouve donc localisé dans une zone 9' d'évacuation du biogaz, située au niveau de la partie supérieure de la cuve 8 du digesteur 7.

Sur la figure 1, n'a été représenté, en coupe, qu'un seul élément de séparation perforé 13 mais, de préférence, une pluralité d'éléments de séparation perforés 13a, 13b, 13c etc. peuvent être montés sur la conduite 14.

De manière alternative, l'élément de séparation perforé supérieur 13 peut être localisé latéralement au niveau de la partie supérieure de la cuve 8 (non représenté dans la figure 1). Dans ce cas, la zone 9' peut correspondre à une excroissance située latéralement sur la partie verticale de la partie supérieure de la cuve 8 et la zone 9 pourrait couvrir la totalité de la surface supérieure du digesteur 7.

La cuve 8 comporte également une tuyauterie 16 qui est en saillie vers l'extérieur par rapport à la surface supérieure 12 de la cuve 8. La tuyauterie 16 est ouverte vers l'extérieur ce qui permet d'acheminer l'air à l'intérieur de la cuve 8 du digesteur 7 ou de l'évacuer vers l'extérieur de la cuve 8. La vanne 17, montée sur la tuyauterie 16, permet de rendre possible ou d'empêcher l'introduction ou l'évacuation de l'air à l'intérieur de la cuve 8. La tuyauterie 16 est localisée dans la zone 9'.

En variante, la tuyauterie 16 peut être située latéralement au niveau de la partie supérieure de la paroi de la cuve 8.

Sur la figure 1 est représenté l'air 24 qui est à la fois localisé à l'extérieur et à l'intérieur de la cuve 8.

La cuve 8 comporte également, au niveau de sa partie inférieure, un élément de séparation perforé inférieur 18, de préférence un plancher perforé occupant partiellement ou totalement le fond de la cuve 8. Ainsi l'élément de séparation perforé inférieur 18 peut correspondre à une couronne perforée occupant partiellement le fond de la cuve 8.

De manière alternative, l'élément de séparation perforé inférieur 18 peut correspondre à un élément vertical situé en surépaisseur de la virole ou de la partie du mur vertical de la cuve 8. L'élément de séparation perforé inférieur 18 peut également correspondre à tout autre type d'élément perforé situé en amont de la tuyauterie 6b, lors des opération de vidange aérobie ou anaérobie du digestat liquide de la cuve 8 du digesteur 7 vers la cuve 3 de l'unité de digestion principale 1.

L'élément de séparation 18 comprend une pluralité d'interstices 18' afin de laisser circuler le digestat liquide 5 lors des étapes de vidanges anaérobies et aérobies du digesteur 7 tout en retenant le digestat solide dans la cuve 8 du digesteur 7.

Dans le mode de réalisation illustré dans la figure 1, la cuve 8 comporte au niveau de ses parois, des résidus de biomasse solide digérée 19, reposant sur l'élément perforé 18 et/ou sur le fond de la cuve 8, qui peuvent être issus d'un précédent cycle de méthanisation.

De manière alternative, au démarrage du cycle de méthanisation, la cuve 8 peut être vide et ne contenir aucune biomasse solide digérée résiduelle au niveau de ses parois.

Sur la figure 1, un grappin 20 est positionné au-dessus de l'orifice d'entrée 9 de la cuve 8 et est suspendu sur un pont roulant 22 par l'intermédiaire d'un câble ou d'un filin pouvant être en acier. Le grappin 20, tel que représenté, comporte une pluralité de crochets 21 et est piloté à partir du pont roulant 22.

En variante, le grappin 20 correspond à une benne ayant une pluralité de godets.

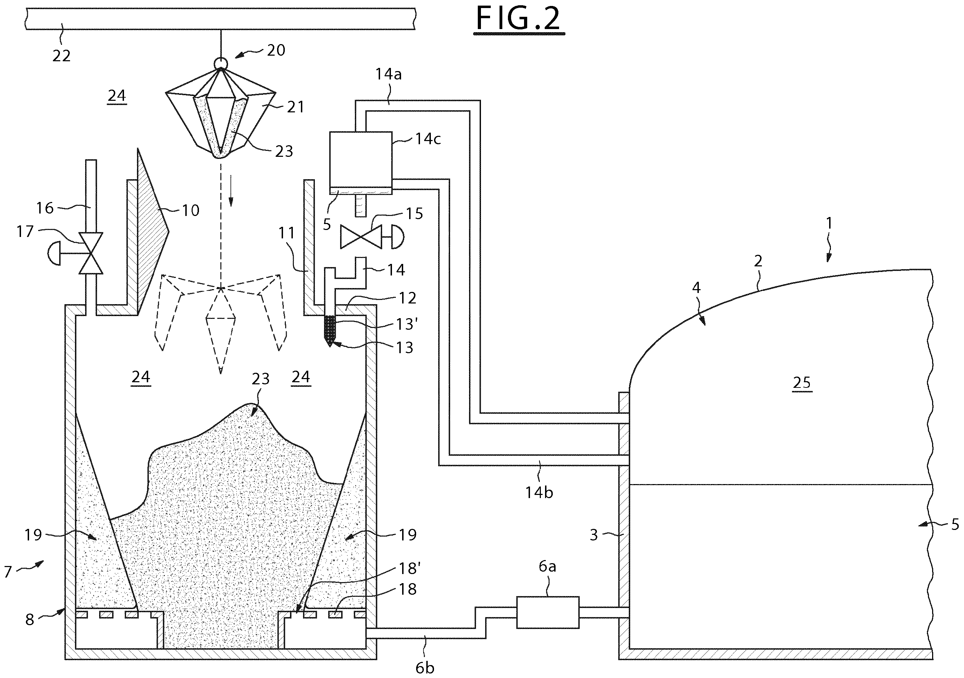

Les figures 2 à 8 représentent les étapes successives d'un procédé de production de biogaz conforme à l'invention.

Sur la figure 2 est représentée schématiquement l'étape de remplissage de la cuve 8 du digesteur 7 par de la biomasse solide 23, en particulier fibreuse et/ou hétérogène, à l'aide du grappin 20.

Au cours de cette étape, le toit amovible 10 est en position relevée, c'est-à-dire en position ouverte. Le grappin 20 permet de saisir la biomasse solide 23 stockée à l'extérieur du digesteur 7 afin de la déposer à l'intérieur de la cuve 8 du digesteur 7. En d'autres termes, le grappin 20 permet d'alimenter, par le haut, la cuve 8 du digesteur 7, en biomasse solide 23, à travers l'orifice d'entrée 9 qui est situé au niveau de la surface supérieure 12 de la cuve 8 du digesteur 7. Ainsi l'orifice d'entrée 9 constitue une zone d'admission de la biomasse solide 23.

Le grappin 20 permet de saisir la biomasse solide 23 contenant éventuellement des éléments indésirables, tels que des cailloux, du gravier, du sable, des barbelés, des ficelles, des pièces métalliques provenant d'outils agricoles ou encore des sacs plastiques, sans avoir besoin d'effectuer d'opération de tri préalable, ni de broyage, ni de dilution de la biomasse solide dans un liquide.

En fonction de son taux de matière sèche, une fraction liquide de la biomasse 23 peut éventuellement couler du grappin au moment de l'étape d'alimentation de la cuve 8.

En variante, le grappin 20 peut également servir à ouvrir le toit amovible 10 pour le déposer à côté de la cuve 8 ou plus loin, et à le refermer par la suite.

La biomasse solide 23 se trouve alors à l'intérieur de la cuve 8 et repose sur le fond et/ou tout ou partie de l'élément de séparation perforé inférieur 18.

Les interstices 18' de l'élément de séparation perforé inférieur 18 présentent une taille suffisamment faible pour éviter que la biomasse 23 ne puisse pénétrer dans la tuyauterie 6b lors des étapes de vidange anaérobies et aérobies de la cuve 8.

L'étape d'alimentation est arrêtée à partir du moment où la quantité en biomasse solide 23 introduite est jugée suffisante pour démarrer la méthanisation.

Lorsque l'étape de remplissage de la cuve 8 par de la biomasse solide 23 est terminée, le toit amovible est fermé de manière à recouvrir complètement de façon étanche l'orifice d'entrée 9.

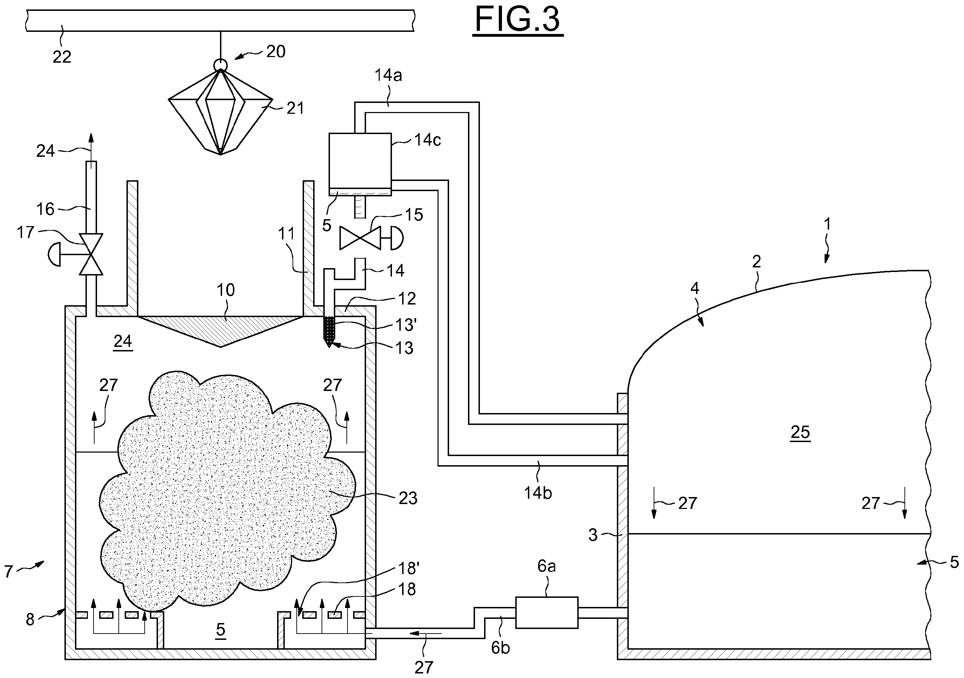

Ainsi la figure 3 illustre la cuve 8 du digesteur 7, après fermeture du toit amovible 10, qui est remplie avec du digestat liquide 5, acheminé par le biais de la pompe d'alimentation 6a, en provenance de la cuve 3 de l'unité de digestion principale 1. En particulier, sur la figure 3, le digestat liquide 5 est acheminé au niveau de la partie inférieure de la cuve 8 sous l'élément perforé 18 et circule à travers les interstices 18' du plancher 18.

La cuve 8 du digesteur 7 peut également être remplie avec du digestat liquide 5 à travers une entrée située dans une zone située au-dessus de l'élément perforé inférieur 18.

Ainsi la cuve 8 peut être remplie avec du digestat liquide 5 qui ne passe pas à travers l'élément perforé inférieur 18.

De manière alternative, le digestat liquide 5 peut être aussi acheminé par une entrée située en tout autre endroit de la cuve 8.

Au cours de cette étape de remplissage de la cuve 8 par du digestat liquide 5, la vanne 17 est laissée en position ouverte de manière à ce que l'air, présent dans la cuve 8, soit évacué du digesteur 7 par l'intermédiaire de la tuyauterie 16. Sur la figure 3 est représenté schématiquement, l'air 24 qui s'échappe de la tuyauterie 16 pendant le remplissage en digestat liquide 5 de la cuve 8. La vanne 15 est fermée de manière à ce que l'air 24 ne circule pas vers l'unité de digestion principale 1.

La biomasse solide 23, et éventuellement la biomasse solide digérée résiduelle 19 issue d'un précédent cycle de méthanisation, se retrouve alors immergée dans le digestat liquide 5 à l'intérieur de la cuve 8. Dans le mode de réalisation illustré dans la figure 3, la biomasse solide 23 et les résidus de biomasse digérée 19 se retrouvent mélangées entre eux.

Le niveau du digestat liquide 5 dans la cuve 3 du digesteur principal 1 diminue tandis qu'il augmente à l'intérieur de la cuve 8 chassant l'air, se trouvant à l'intérieur de la cuve 8, qui s'échappe à travers la tuyauterie 16. L'étape de remplissage sera considérée comme étant terminée lorsque le niveau du digestat liquide 5 aura atteint et légèrement dépassé vers le haut le niveau de la vanne 17, et que la totalité de l'air contenu dans la cuve 8 aura été évacuée.

Sur la figure 3 est représenté, d'une part, le sens de circulation 27 du digestat liquide 5 à l'intérieur de la cuve 8 du digesteur 7 qui reflète l'augmentation de niveau dans la cuve 8 et, d'autre part, le sens de circulation 27 qui illustre la baisse de niveau du digestat liquide 5 à l'intérieur de l'unité de digestion principale 1.

De plus, le sens de circulation 27 du digestat liquide 5 est représenté à l'intérieur de la tuyauterie 6b.

Conformément à la figure 4, une fois la cuve 8 remplie par le digestat liquide 5, la vanne 17 est fermée de manière à placer le digesteur 7 dans des conditions anaérobies, c'est-à-dire à empêcher toute introduction d'air dans la cuve 8, tandis que la vanne 15 est ouverte afin de recueillir le mélange biogaz et digestat liquide.

La figure 4 représente donc l'étape de digestion de la biomasse 23 dans des conditions anaérobies. Au cours de cette étape, du biogaz se forme au sein de la cuve 8.

Une percolation du digestat liquide 5 à travers la biomasse 23 est mise en œuvre par le pompage du digestat liquide 5 en provenance de l'unité de digestion principale 1. La percolation se déroule à travers la biomasse solide 23 de manière à ce que la plus grande partie possible de la biomasse solide 23 soit mise en contact intime avec les bactéries contenues dans le digestat liquide 5 et puisse ainsi réagir au cours de la digestion. Dans la figure 4 est représenté schématiquement le flux de digestat liquide 5 circulant, par percolation verticale ascendante, à travers l'ensemble de la biomasse solide 23.

Conformément à la figure 4, de préférence, l'alimentation en digestat liquide 5 continue au cours de toute l'étape de digestion afin que le digestat liquide 5 continue de circuler à travers la biomasse solide 23, ce qui diminue le risque de survenue de réactions chimiques ou biologiques ponctuelles indésirables en certains endroits du tas de biomasse solide 23, tel que l'acidose par exemple, lesquelles pourraient altérer la qualité de la flore bactérienne.

Cependant, l'alimentation en digestat liquide 5 peut être, dans certains cas, temporairement ou définitivement interrompue, en particulier vers la fin du cycle de méthanisation, moment où les réactions chimiques et biologiques sont moins virulentes.

Au cours de cette étape, un processus de diffusion du digestat liquide 5 dans la biomasse solide 23 se produit. Un tel processus de diffusion est favorisée, d'une part, par l'immersion de la biomasse solide 23 dans le digestat liquide 5 et, d'autre part, par la pression du digestat liquide 5 sur la biomasse solide 23 qui résulte de l'action de la pompe 6a.

Dans le mode de réalisation de la figure 4, la biomasse fibreuse solide 23 se désagrège au cours de la percolation du digestat liquide 5 à travers la biomasse solide 23 et de la diffusion du digestat liquide 5 dans la biomasse solide 23.

Le biogaz 25 et le digestat liquide 5 sont extraits à travers les interstices 13' de l'élément de séparation perforé supérieur 13 tandis que la biomasse solide 23 reste immergée dans la cuve 8. Le mélange de biogaz 25 et de digestat liquide 5, circulant à travers l'élément de séparation perforé supérieur 13, est représenté schématiquement sur la figure 4.

Le biogaz et le digestat liquide sont acheminés ensemble dans le circuit 14 jusqu'au pot de séparation 14c. Dans la figure 4, après séparation du biogaz 25 et du digestat liquide 5, la tuyauterie 14a achemine principalement le biogaz 25 tandis que la tuyauterie 14b achemine principalement le digestat liquide 5.

Sur la figure 4 est représenté le niveau 5a du digestat liquide 5 dans le pot de séparation 14c. Le digestat liquide 5 se retrouve acheminé dans un circuit d'acheminement 14b correspondant à une conduite de débordement du digestat liquide. Le digestat liquide 5 présent dans le circuit 14b est acheminé jusqu'à la cuve 3, remplie de digestat liquide 5, du digesteur principal 1. Dans la figure 4, le digestat liquide 5 injecté dans la cuve 8 du digesteur 7, par le biais de la pompe 6, est recirculé par débordement, au moyen du circuit d'acheminement 14b, dans la cuve 3 du digesteur principal 1. Le sens de circulation du digestat liquide 5 est représenté par les flèches 27.

Optionnellement, la présence d'une pompe de circulation (non représentée sur la figure 4) montée sur le circuit 14b permet d'aspirer le digestat liquide 5 à travers l'élément de séparation perforé supérieur 13.

Le biogaz 25 est quant à lui acheminé dans le circuit 14a jusqu'au digesteur principal 1, en particulier à un niveau proche de la membrane étanche 2, lequel niveau est de préférence supérieur au niveau maximal de stockage du digestat liquide 5 dans la cuve 3 de l'unité de digestion principale 1.

Ainsi l'élément de séparation perforé supérieur 13 est localisé dans une zone d'évacuation 9' du biogaz 25 qui est situé au niveau de la partie supérieure de la cuve 8 de digestion.

Autrement dit, la zone 9' constitue l'espace d'évacuation du digestat liquide 5 et du biogaz 25.

De plus, la zone 9' constitue l'espace d'évacuation ou d'introduction de l'air dans la cuve 8.

L'étape de digestion, permet de produire du biogaz sans avoir à mettre nécessairement en œuvre une étape de brassage de la biomasse solide 23 ce qui permet de diminuer les investissements en matériels de brassage et d'agitation et de réduire les dépenses énergétiques et de maintenance généralement afférentes à cette étape.

Conformément à la figure 4, un bouchon 26 peut être positionné sur le toit amovible 10 de la cuve 8 de manière à exercer une pression afin de mieux assurer son étanchéité et sa bonne fermeture au cours de l'étape de digestion.

Le bouchon 26 peut être un bouchon liquide constitué d'eau claire ou de digestat liquide pouvant provenir de la réserve en digestat liquide 5 de l'unité de digestion principale 1 et/ou de fuites dans le toit 10 en provenance de la cuve 8. Le bouchon 26 pourra être recouvert par le biais d'un couvercle, d'un toit fermé à double paroi ou d'un couvercle en plastique, notamment une bâche en plastique. De préférence, le niveau supérieur du bouchon liquide 26 sera le même que le niveau de débordement du digestat liquide 5 dans le pot de séparation 14c, vers la tuyauterie de circulation 14b.

En variante, la séparation du biogaz 25 et du digestat liquide 5 peut s'effectuer sans la présence d'un pot de séparation 14c, voire sans la présence de plusieurs tuyauteries d'acheminement 14a ou 14b. Dans ce cas, la séparation du biogaz 25 et du digestat liquide 5 aurait lieu dans l'unité de digestion principale 1.

Durant l'étape de digestion, la biomasse solide 23 est immergée dans le digestat liquide 5 et peut en partie flotter en haut de la cuve 8, se trouver en partie en suspension au milieu de la cuve 8 ou en partie décanter et se trouver en bas de la cuve 8.

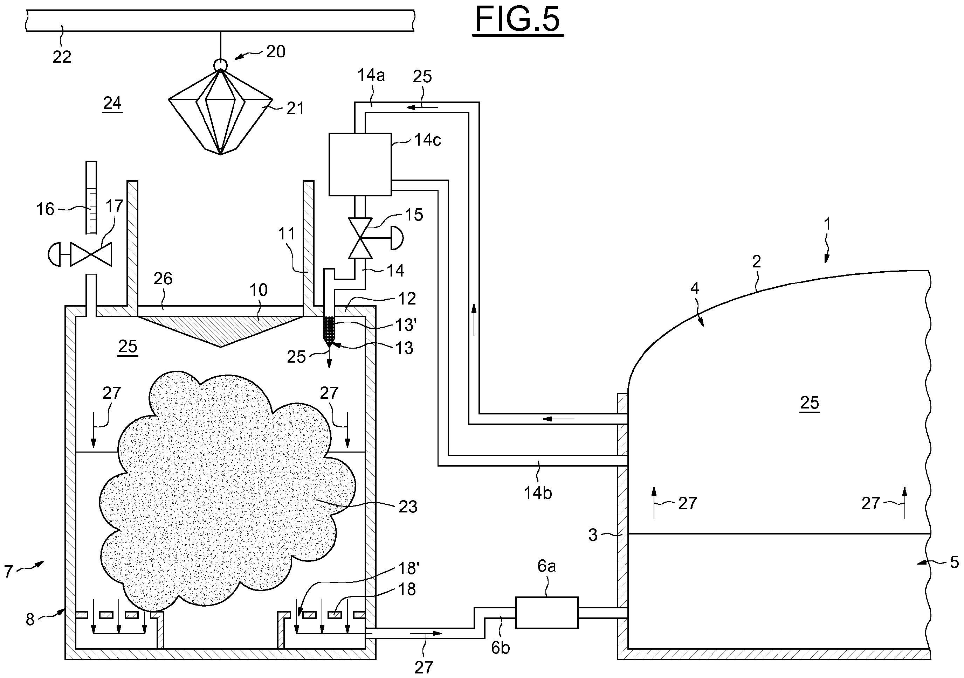

Sur la figure 5 est représentée schématiquement l'étape de vidange anaérobie du digestat liquide 5 de la cuve 8 du digesteur 7, vers la cuve 3 de l'unité de digestion principale 1, en vidangeant le digestat liquide 5 par l'intermédiaire de la pompe 6a et en conservant la vanne 17 en position fermée tandis que la vanne 15 est en position ouverte.

L'étape de vidange permet, par le tassement au fond du digesteur 7 de la biomasse solide 23 partiellement digérée suite à l'étape de digestion et de diffusion, de supprimer les éventuels chemins préférentiels de percolation ainsi que les éventuelles poches de biogaz, bloquées dans la biomasse 23. Ainsi le biogaz 25, se trouvant dans le circuit 14a, circule depuis l'unité de digestion principale 1 en direction de l'intérieur de la cuve 8 du digesteur 7 en passant à travers l'élément de séparation perforé supérieur 13. Le biogaz vient ainsi remplir l'espace laissé vacant par la vidange du digestat liquide 5. Au cours de cette étape de vidange, la biomasse solide 23 redescend vers le fond de la cuve 8 en direction de l'élément de séparation perforé inférieur 18, et vient finalement se déposer sur le fond de la cuve 8 et/ou sur l'élément de séparation perforé inférieur 18.

Sur la figure 5 est représenté le sens de circulation du biogaz 25 dans le circuit 14 en direction de la cuve 8 ainsi que le sens de circulation 27 du digestat liquide 5 en direction de la cuve 3 de l'unité de digestion principale 1.

L'élément de séparation perforé inférieur 18 présente l'avantage de laisser passer le digestat liquide 5 au moment de la vidange anaérobie en retenant dans la cuve 8 la biomasse solide 23 partiellement digéré. L'élément de séparation perforé inférieur 18 permet donc de séparer le digestat liquide 5, en le laissant circuler, et la biomasse solide 23 ce qui permet de ne pas risquer d'obstruer la pompe d'alimentation 6a et de ne pas introduire du digestat solide 23 dans l'unité de digestion principale 1.

Durant l'étape de vidange anaérobie, le bouchon liquide 26 peut être vidangé partiellement ou totalement, notamment pour éviter que s'exerce une pression trop forte sur le toit 10. La partie liquide évacuée peut être vidangée à l'intérieur de la cuve 8 du digesteur 7, ou bien être stockée dans un lieu de stockage pour un usage ultérieur, ou encore être transférée vers une autre cuve de digestion, ou enfin être évacuée vers toute autre destination.

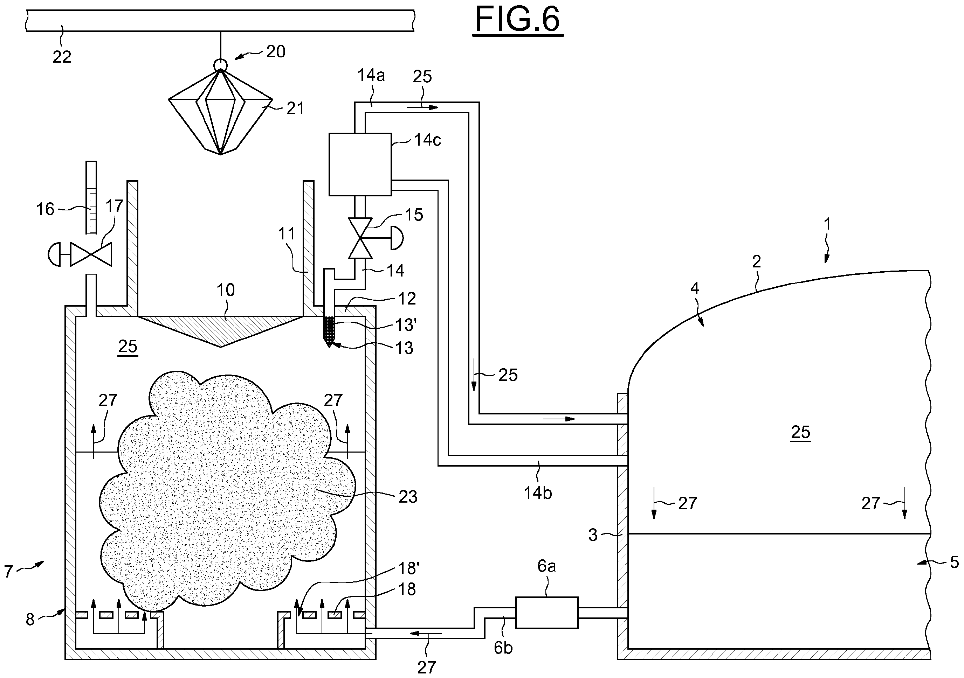

Après la vidange anaérobie de la cuve 8, une nouvelle étape de remplissage de la cuve 8 avec du digestat liquide 5 est mise en œuvre comme représenté sur la figure 6. L'étape de remplissage est identique à la précédente étape de remplissage à l'exception du fait que la vanne 17 est en position fermée et que la vanne 15 est en position ouverte.

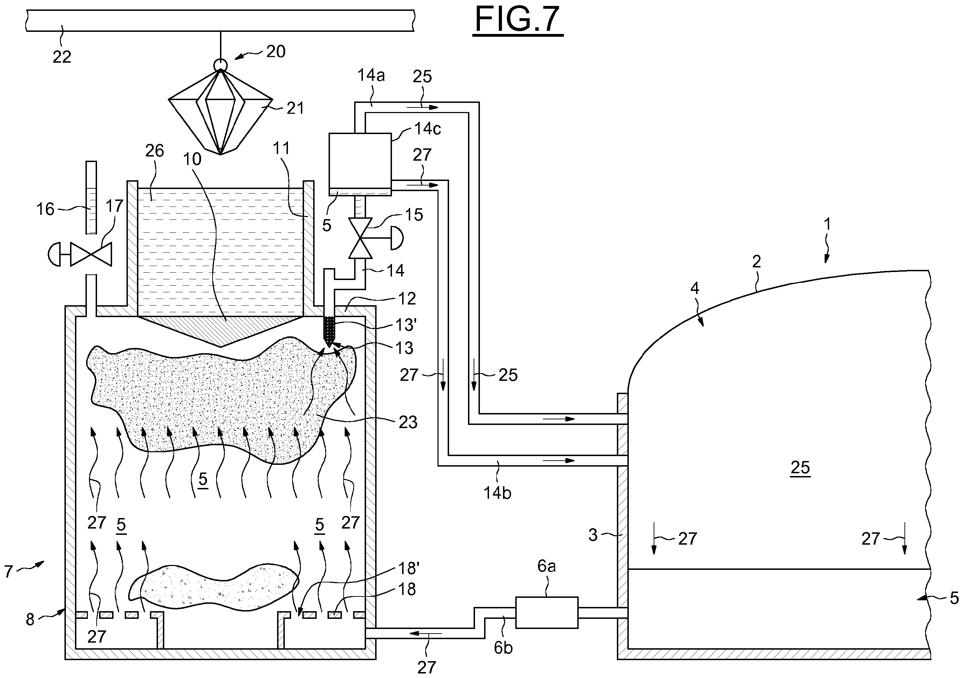

Conformément à la figure 7, une fois l'étape de remplissage terminée, l'étape de digestion de la biomasse solide 23 est une nouvelle fois mise en œuvre afin de continuer la production de biogaz 25.

De la même façon que précédemment, le biogaz 25, issu de l'étape de digestion, est principalement acheminé par le biais du circuit d'acheminement 14a jusqu'à l'unité de digestion principale 1.

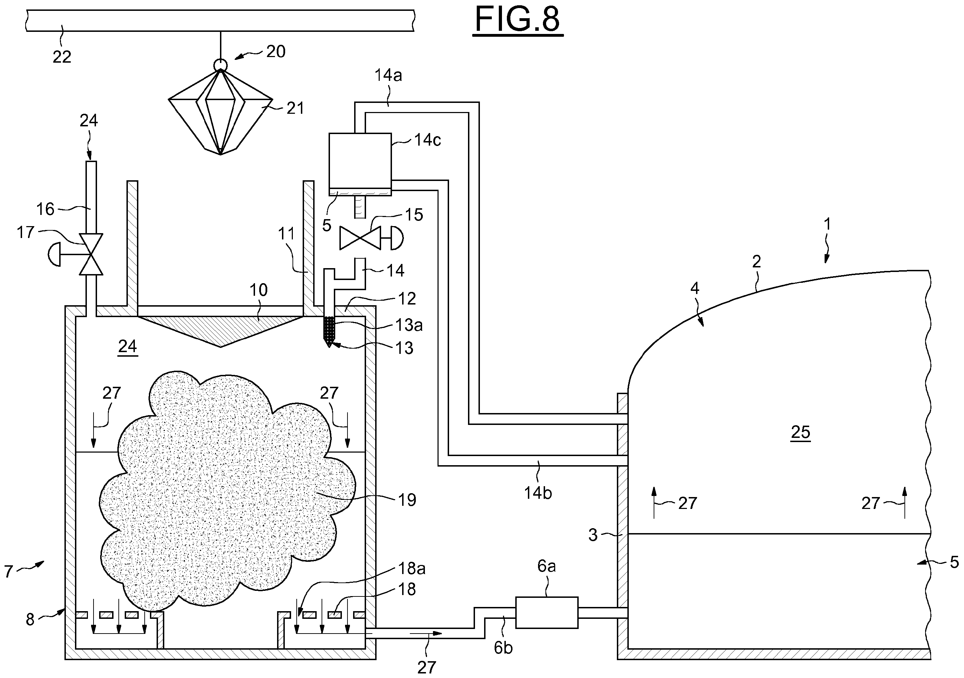

Une fois l'étape de digestion terminée, c'est-à-dire en fin de cycle au bout d'environ 20 à 80 jours, une étape de vidange aérobie de la cuve 8 du digesteur 7 est mise en œuvre comme illustré sur la figure 8.

L'étape de vidange aérobie est mise en œuvre en fermant la vanne 15 afin d'empêcher toute introduction de biogaz 25 à l'intérieur de la cuve 8 de digestion, et en ouvrant la vanne 17 afin d'introduire l'air 24 à l'intérieur de la cuve 8 de digestion en remplacement du digestat liquide 5 évacué et en pompant le digestat liquide 5 par le biais de la pompe 6a à travers l'élément de séparation perforé inférieur 18 afin d'évacuer le digestat liquide 5 vers la cuve 3 de l'unité de digestion principale 1.

Cette étape permet de vidanger le digestat liquide de la cuve 8 de digestion en évitant la présence de biogaz dans la cuve 8, ce qui permet de minimiser les fuites/rejets de biogaz dans l'atmosphère au moment de la mise en œuvre ultérieure de l'étape d'évacuation de la biomasse solide 19 résiduelle après digestion, c'est-à-dire le digestat solide.

Sur cette figure, le niveau du digestat liquide 5 diminue et l'air 24 s'introduit progressivement à l'intérieur de la cuve 8 du digesteur 7 en comblant l'espace laissé vacant par la vidange du digestat liquide 5.

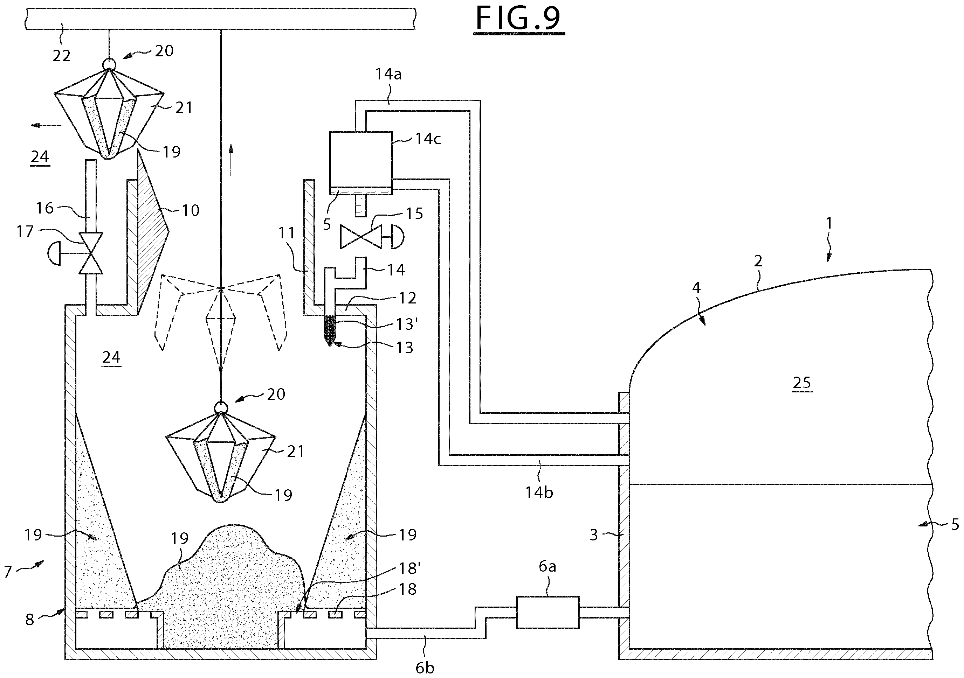

La figure 9 décrit l'étape d'évacuation de la biomasse solide digérée résiduelle 19, une fois la digestion terminée, par le grappin 20. En particulier, le toit amovible 10 est relevé de manière à ce que le grappin 20 puisse être actionné à travers l'orifice d'entrée 9 de la cuve 8. De cette façon, le grappin 20 permet de saisir la biomasse solide digérée résiduelle 19, une fois la digestion terminée, de manière à l'évacuer soit vers une zone de stockage, une zone de traitement ou un engin de transport.

Dans le mode de réalisation illustré sur la figure 9, le grappin 20 saisit la biomasse solide digérée résiduelle 19 dans la cuve 8 du digesteur 7 en laissant éventuellement subsister des résidus de biomasse solide digérée 19 au niveau des parois ou du fond de la cuve 8.

Des résidus de biomasse solide digérée 19 peuvent être laissés au niveau des parois, ou dans le fond de la cuve 8, ou encore sur l'élément de séparation inférieur 18, car ils peuvent constituer un bon substrat, très riche en bactéries qui seront susceptibles d'agir lors du prochain cycle de méthanisation.

Grâce à l'étape de vidange aérobie précédente, l'étape de récupération de la biomasse solide digérée résiduelle 19 après méthanisation, correspondant au digestat solide, peut se dérouler en toute sécurité en minimisant les risques de rejet de biogaz dans l'atmosphère ainsi que les risques de mise en contact avec le biogaz des personnes, le grappin et des équipements situés en dehors de la cuve 8 de digestion.

Ainsi l'orifice d'entrée 9 de la cuve 8 représente à la fois une zone d'admission de la biomasse solide 23 avant méthanisation ainsi qu'une zone d'évacuation de la biomasse solide digérée résiduelle 19 après méthanisation, appelée digestat solide

En d'autres termes, l'orifice d'entrée 9 de la cuve 8 constitue un espace d'introduction de la biomasse solide et d'évacuation de la biomasse solide digérée résiduelle.

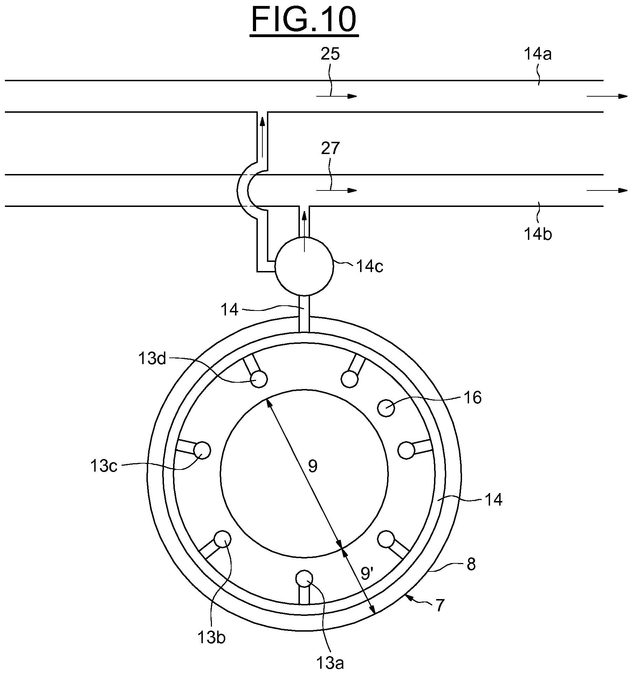

Sur la figure 10 est représentée de manière schématique une vue de dessus de la cuve 8 du digesteur 7, selon l'invention, comprenant un orifice d'entrée 9 constituant une zone d'admission de la biomasse solide 23 et un zone d'évacuation de la biomasse solide digérée résiduelle 19 après réaction de méthanisation.

Le digesteur 7 présente également une zone 9' d'évacuation du biogaz 25 et d'introduction et d'évacuation d'air.

La partie supérieure de la cuve 8, dans son espace d'évacuation du biogaz 9', est dotée de plusieurs éléments de séparation perforés supérieurs 13a, 13b, 13c et 13d, de préférence des tuyaux perforés, qui sont en saillie s'étendant de l'intérieur jusqu'au bord supérieur de la cuve 8.

Les éléments de séparation perforés supérieurs 13a, 13b, 13c, et 13d sont reliés à une tuyauterie 14 comportant un pot de séparation 14c connecté à deux circuits distincts 14a et 14b qui sont reliés à l'unité de digestion principale 1 (non représentée sur la figure 10).

Le circuit 14a constitue une conduite générale acheminant principalement du biogaz et le circuit 14b constitue une conduite générale acheminant principalement du digestat liquide.

Comme indiqué précédemment, au cours de l'étape de digestion, un mélange de biogaz 25 et de digestat liquide 5 est extrait à travers les éléments de séparation perforés supérieurs 13a, 13b, 13c, 13d et est acheminé dans la tuyauterie 14.

Le digestat liquide 5 se retrouve ensuite acheminé principalement dans le circuit 14b vers la réserve en digestat liquide 5 d'une unité de digestion principale 1 et le biogaz 25 se retrouve acheminé principalement dans le circuit 14a vers l'espace de stockage de gaz 4 de l'unité de digestion principale 1.

La partie supérieure de la cuve 8, dans son espace d'évacuation du biogaz 9', est aussi dotée d'une ou plusieurs ouvertures 16, permettant l'introduction ou l'évacuation de l'air durant certaines phases de chargement aérobie du digestat liquide ou de déchargement aérobie du digestat liquide 5.

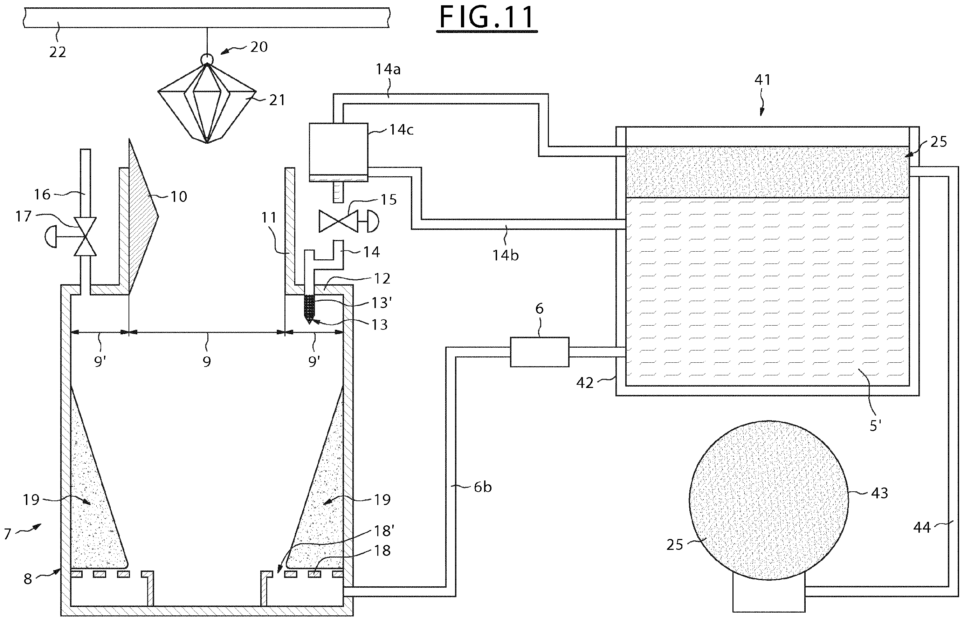

Sur la figure 11 est représenté schématiquement un digesteur 7, selon l'invention, relié à une unité de digestion principale composée de deux ouvrages distincts.

Conformément à cette figure, l'unité de digestion principale comprend un premier ouvrage 41, qui correspond à une cuve 42 contenant du digestat liquide 5 surmontée en surface par une faible quantité en biogaz 25. Le deuxième ouvrage 43 correspond à un gazomètre apte à stocker du biogaz 25.

Les deux ouvrages 41 et 43 sont reliés entre eux par une tuyauterie 44.

Ainsi l'unité de digestion principale est apte à stocker séparément principalement du biogaz 25 et principalement du digestat liquide 5.

Sur la figure 11, le circuit d'acheminent 14 comporte un pot de séparation 14c relié à deux circuits distincts 14a et 14b qui sont reliés à l'unité de digestion principale. En particulier, le circuit d'acheminement 14b est relié à la cuve 42 au niveau de la réserve en digestat liquide 5 et le circuit d'acheminement 14a est connecté à la partie supérieure de l'ouvrage 42 dans une zone ne comportant pas de digestat liquide 5. En alternative, le circuit d'acheminement 14b peut être connecté à la partie supérieure de l'ouvrage 42 dans une zone ne comportant pas de digestat liquide 5.

Comme indiqué précédemment, au cours de l'étape de digestion, un mélange de biogaz 5 et de digestat liquide 25 est extrait à travers l'élément de séparation perforé supérieur 13' et est acheminé dans la tuyauterie 14.

Le digestat liquide se retrouve alors acheminé principalement dans le circuit 14b vers la réserve en digestat liquide 5 de la cuve 42.

Le biogaz 5 quant à lui circule principalement dans le circuit 14a et traverse l'ouvrage 41, dans une zone ne comportant pas de digestat liquide 5, pour être acheminé, par le biais de la tuyauterie 44, vers le gazomètre 43.

De cette façon, l'ouvrage 41 comporte, en surface du digestat liquide 25, une faible quantité de biogaz 5 provenant du circuit 14a reliant le digesteur 7 et l'unité de digestion principale 41.

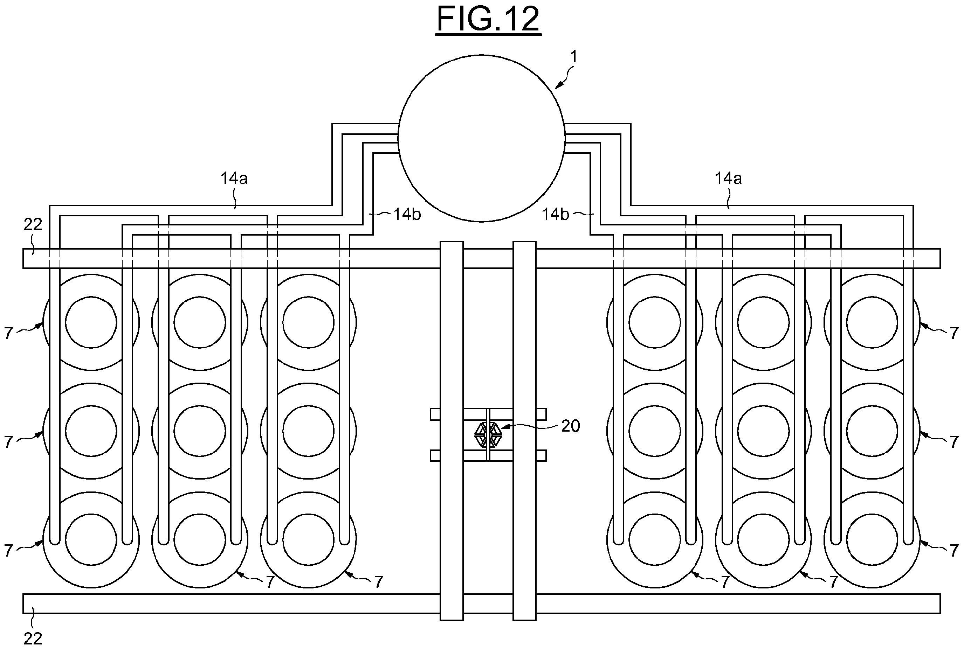

La figure 12 représente schématiquement une vue de dessus d'un système ou d'une installation de production de biogaz comprenant l'unité de digestion principale 1 susceptible d'être alimentée par débordement en digestat liquide, via un circuit d'acheminement 14b, par une pluralité de digesteurs 7 alimentés par le haut en biomasse solide par le biais d'un grappin 20 monté sur un pont roulant 22.

L'unité de digestion principale 1 est également alimentée en biogaz par le biais d'un circuit d'acheminement 14a suite à la production de biogaz dans chaque digesteur 7.

L'unité de digestion principale 1 n'est pas, ou très faiblement, alimentée en biomasse solide 23 au cours du procédé de méthanisation mais constitue avantageusement une réserve en digestat liquide 5 et un espace de stockage en biogaz 25.

La réserve en digestat liquide 5 sert donc à alimenter les digesteurs 7 et l'espace de stockage en biogaz 25 sert à récupérer le biogaz 25 issu des différents digesteurs 7.

Au cours du procédé de méthanisation considéré dans son ensemble, le grappin 20 circule à travers les différents digesteurs 7 afin de les alimenter par le haut avec de la biomasse solide 23.

De la même façon, le grappin 20 circule à travers les différents digesteurs 7 afin de récupérer par le haut les biomasses solides digérées résiduelles 19, ou digestats solides, en fin de cycle de méthanisation.

## Revendications

1. Système de production de biogaz (25) comportant :
- au moins une unité de digestion principale (1), constitué d'un ou plusieurs ouvrages, apte à contenir du biogaz (25) et du digestat liquide (5),
- au moins un digesteur de méthanisation (7), destiné à la production de biogaz (25), comportant une cuve (8), apte à contenir de la biomasse solide (23), et comportant au niveau de sa partie supérieure : au moins une zone (9) d'admission de la biomasse solide (23) et d'évacuation de la biomasse solide digérée résiduelle (19), sur laquelle un toit (10) est apte à s'ouvrir et à se refermer, et au moins une zone (9') d'évacuation du biogaz (25) ;
- ladite zone d'évacuation du biogaz (9') comprenant au moins un élément de séparation perforé supérieur (13, 13a, 13b, 13c, 13d), situé au niveau de la partie supérieure de la cuve, apte à séparer, d'une part, un mélange composé de biogaz (25) et de digestat liquide (5) et, d'autre part, de la biomasse solide (23),
- ledit digesteur de méthanisation (7) étant surmonté par un moyen de préhension (20) apte à permettre l'alimentation de la biomasse solide (23) et l'évacuation de la biomasse solide digérée résiduelle (19),
- ledit digesteur étant relié, par le biais d'un circuit d'alimentation (6b) en digestat liquide (5) et par le biais d'un circuit d'évacuation (14) de biogaz (25) et de digestat liquide (5) à ladite unité de digestion principale (1).

2. Système selon la revendication 1, **caractérisé en ce que** le moyen de préhension (20) est un grappin (21) comportant une pluralité de crochets ou une benne comportant des godets.

3. Système selon la revendication 1 ou 2, **caractérisé en ce que** l'élément de séparation perforé supérieur (13, 13a, 13b, 13c, 13d) est un tuyau perforé dont l'extrémité est obturée partiellement ou totalement.

4. Système selon l'une quelconque des revendications précédentes, **caractérisé en ce que** ledit digesteur (7) comprend au moins un élément de séparation perforé inférieur (18), situé au niveau de la partie inférieure de la cuve (8), apte à séparer le digestat liquide (5) de la biomasse solide (23).

5. Système selon la revendication 4, **caractérisée en ce que** l'élément de séparation perforé inférieur (18) est un plancher perforé.

6. Système selon l'une quelconque des revendications précédentes, **caractérisé en ce que** ledit digesteur (7) comprend une ouverture (16) munie d'une vanne (17), apte à obturer ou permettre le passage de l'air (24) entre l'intérieur et l'extérieur de la cuve (8) du digesteur (7) et une vanne (15), située sur un circuit d'acheminement (14), apte à obturer ou permettre la circulation de biogaz (25) et de digestat liquide (5) entre le digesteur (7) et une unité de digestion principale (1) telle que défini selon la revendication 1.

7. Système selon l'une quelconque des revendications précédentes, **caractérisé en ce que** ledit digesteur (7) comporte un bouchon liquide (26) positionné sur le toit (10) de la cuve (8).

8. Système selon l'une quelconque des revendications précédentes, **caractérisé en ce que** l'unité de digestion principale (1) est constituée par deux ouvrages distincts, l'un destiné à contenir principalement du digestat liquide (5), l'autre destiné à contenir principalement du biogaz (25).

9. Système selon l'une quelconque des revendications précédentes, **caractérisé en ce que** le circuit d'évacuation (14) comprend un circuit (14a) apte à acheminer du biogaz (25) et un circuit (14b) apte à acheminer du digestat liquide (25).

10. Procédé de production de biogaz (25) comprenant au moins les étapes successives suivantes :
- une étape d'alimentation en biomasse solide (23), mise en œuvre par au moins un moyen de préhension (20), à travers une zone (9) d'admission et d'évacuation située au niveau de la partie supérieure d'une cuve (8) d'un digesteur (7), tel que défini dans la revendication 1, et sur laquelle un toit (10) est apte à s'ouvrir et à se refermer,
- une étape de remplissage de la cuve (8) du digesteur (7) avec un digestat liquide (5) pour immerger la biomasse solide (23),
- une étape de digestion, constituée d'une percolation du digestat liquide (5) à travers la biomasse solide (23) et d'une diffusion du digestat liquide (5) dans la biomasse solide (23) dans des conditions anaérobies pour générer puis récupérer le biogaz (25) à travers une zone d'évacuation (9'), située dans la partie supérieure dudit digesteur (7), et
- une étape de vidange aérobie du digestat liquide (5) de la cuve (8) du digesteur (7), et
- une étape d'évacuation de la biomasse solide digérée résiduelle (19), mise en oeuvre par au moins un moyen de préhension (20), à travers la zone (9) du digesteur (7).

11. Procédé de production de biogaz (25) selon la revendication 10, **caractérisé en ce qu'**il comprend en outre entre l'étape de digestion et l'étape de vidange aérobie du digestat liquide (5) au moins les étapes suivantes :
- une étape de vidange anaérobie du digestat liquide (5) de la cuve (8) du digesteur (7), et
- une étape de re-remplissage de la cuve (8) du digesteur (7) avec un digestat liquide (5) après vidange anaérobie.

12. Procédé de production de biogaz (25), selon la revendication 11, **caractérisé en ce qu'**il comprend en outre, après l'étape de re-remplissage de la cuve (8) dudit digesteur (7) avec un digestat liquide (5), au moins une étape de digestion, constituée d'une percolation du digestat liquide (5) à travers la biomasse solide (23) et d'une diffusion du digestat liquide (5) dans la biomasse solide (23) pour générer puis récupérer le biogaz (25) à travers la zone (9').

## Patentansprüche

1. System zur Herstellung von Biogas (25), aufweisend:
- mindestens eine Hauptgärungseinheit (1), die von einem oder mehreren Elementen gebildet ist, die geeignet ist, Biogas (25) und flüssigen Gärrest (5) zu enthalten,
- mindestens einen Methangärungsbehälter (7) der zur Herstellung von Biogas (25) bestimmt ist, aufweisend einen Tank (8), der geeignet ist, feste Biomasse (23) zu enthalten, und aufweisend an seinem oberen Teilbereich: mindestens einen Bereich (9) zum Einlass der festen Biomasse (23) und zum Ablass der restlichen vergärten festen Biomasse (19), auf dem sich ein Dach (10) öffnen und wieder schließen kann, und mindestens einen Bereich (9') zum Ablass des Biogases (25);
- wobei der Bereich zum Ablass des Biogases (9') mindestens ein oberes perforiertes Trennelement (13, 13a, 13b, 13c, 13d) umfasst, das sich am oberen Teilbereich des Tanks befindet, das geeignet ist, einerseits eine Mischung, die aus Biogas (25) und flüssigem Gärrest (5) gebildet ist, und andererseits feste Biomasse (23) zu trennen,
- wobei der Methangärungsbehälter (7) von einem Greifmittel (20) überragt ist, das geeignet ist, die Versorgung der festen Biomasse (23) und den Ablass der restlichen vergärten festen Biomasse (19) zu gestatten,
- wobei der Gärungsbehälter über eine Leitung zur Versorgung (6b) mit flüssigem Gärrest (5) und über eine Leitung zum Ablass (14) von Biogas (25) und flüssigem Gärrest (5) mit der Hauptgärungseinheit (1) verbunden ist.

2. System nach Anspruch 1, **dadurch gekennzeichnet, dass** das Greifmittel (20) ein Greifer (21) ist, der eine Mehrzahl von Haken oder einen Greifer mit Schaufeln aufweist.

3. System nach Anspruch 1 oder 2, **dadurch gekennzeichnet, dass** das obere perforierte Trennelement (13, 13a, 13b, 13c, 13d) eine perforierte Leitung ist, deren Ende teilweise oder vollständig verschlossen ist.

4. System nach einem der vorhergehenden Ansprüche, **dadurch gekennzeichnet, dass** der Gärungsbehälter (7) mindestens ein unteres perforiertes Trennelement (18) umfasst, das sich am unteren Teilbereich des Tanks (8) befindet, das geeignet ist, den flüssigen Gärrest (5) von der festen Biomasse (23) zu trennen.

5. System nach Anspruch 4, **dadurch gekennzeichnet, dass** das untere perforierte Trennelement (18) ein perforierter Boden ist.

6. System nach einem der vorhergehenden Ansprüche, **dadurch gekennzeichnet, dass** der Gärungsbehälter (7) eine Öffnung (16) umfasst, die mit einem Ventil (17) versehen ist, das geeignet ist, den Durchtritt der Luft (24) zwischen dem Inneren und dem Äußeren des Tanks (8) des Gärungsbehälters (7) zu verhindern oder zu gestatten, und ein Ventil (15), das sich auf einer Beförderungsleitung (14) befindet, das geeignet ist, die Zirkulation von Biogas (25) und flüssigem Gärrest (5) zwischen dem Gärungsbehälter (7) und einer Hauptgärungseinheit (1) nach Anspruch 1 zu verhindern oder zu gestatten.

7. System nach einem der vorhergehenden Ansprüche, **dadurch gekennzeichnet, dass** der Gärungsbehälter (7) einen Flüssigkeitsstopfen (26) aufweist, der auf dem Dach (10) des Tanks (8) positioniert ist.

8. System nach einem der vorhergehenden Ansprüche, **dadurch gekennzeichnet, dass** die Hauptgärungseinheit (1) von zwei separaten Elementen gebildet ist, wobei eins dazu bestimmt ist, hauptsächlich flüssigen Gärrest (5) zu enthalten, wobei das andere dazu bestimmt ist, hauptsächlich Biogas (25) zu enthalten.

9. System nach einem der vorhergehenden Ansprüche, **dadurch gekennzeichnet, dass** die Ablassleitung (14) eine Leitung (14a), die geeignet ist, Biogas (25) zu befördern, und eine Leitung (14b), die geeignet ist, flüssigen Gärrest (25) zu befördern, umfasst.

10. Verfahren zur Herstellung von Biogas (25), umfassend zumindest die folgenden aufeinanderfolgenden Schritte:
- einen Schritt des Versorgens mit fester Biomasse (23), der von mindestens einem Greifmittel (20) ausgeführt wird, durch einen Ein- und Ablassbereich (9), der sich an dem oberen Teilbereich eines Tanks (8) eines Gärungsbehälters (7) nach Anspruch 1 befindet, und auf dem sich ein Dach (10) öffnen und wieder schließen kann,
- einen Schritt des Befüllens des Tanks (8) des Gärungsbehälters (7) mit einem flüssigen Gärrest (5), um die feste Biomasse (23) einzutauchen,
- einen Schritt des Vergärens, der von einem Durchsickern des flüssigen Gärrests (5) durch die feste Biomasse (23) und einem Diffundieren des flüssigen Gärrests (5) in die feste Biomasse (23) unter anaeroben Bedingungen gebildet ist, um das Biogas (25) zu erzeugen und es dann durch einen Ablassbereich (9') zu sammeln, der sich im oberen Teilbereich des Gärungsbehälters (7) befindet, und
- einen Schritt des aeroben Entleerens des flüssigen Gärrests (5) aus dem Tank (8) des Gärungsbehälters (7), und
- einen Schritt des Ablassens der restlichen vergärten festen Biomasse (19), der von mindestens einem Greifmittel (20) durchgeführt wird, durch den Bereich (9) des Gärungsbehälters (7).

11. Verfahren zur Herstellung von Biogas (25) nach Anspruch 10, **dadurch gekennzeichnet, dass** es ferner zwischen dem Schritt des Vergärens und dem Schritt des aeroben Entleerens des flüssigen Gärrests (5) zumindest die folgenden Schritte umfasst:
- einen Schritt des anaeroben Entleerens des flüssigen Gärrests (5) aus dem Tank (8) des Gärungsbehälters (7), und
- einen Schritt des erneuten Befüllens des Tanks (8) des Gärungsbehälters (7) mit einem flüssigen Gärrest (5) nach dem anaeroben Entleeren.

12. Verfahren zur Herstellung von Biogas (25) nach Anspruch 11, **dadurch gekennzeichnet, dass** es ferner nach dem Schritt des erneuten Befüllens des Tanks (8) des Gärungsbehälters (7) mit einem flüssigen Gärrest (5) mindestens einen Schritt des Vergärens umfasst, der von einem Durchsickern des flüssigen Gärrests (5) durch die feste Biomasse (23) und einem Diffundieren des flüssigen Gärrests (5) in die feste Biomasse (23) gebildet ist, um das Biogas (25) zu erzeugen und es dann durch den Bereich (9') zu sammeln.

## Claims

1. A system for production of biogas (25), comprising;
- at least one main digestion unit (1) constituted by one or a plurality of elements which can contain biogas (25) and liquid digestate (5),
- at least one methanization digester (7), which is designed for the production of biogas (25), comprising a tank (8) which can contain solid biomass (23), and comprising in its upper part: at least one area (9) for intake of the solid biomass (23) and discharge of the residual digested solid biomass (19), on which a roof (10) can be opened and closed, and at least one area (9') for discharge of the biogas (25);
- said area for discharge of the biogas (9') comprising at least one upper perforated separation element (13, 13a, 13b, 13c, 13d) situated at the upper part of the tank, which can separate firstly a mixture constituted by biogas (25) and liquid digestate (5), and secondly solid biomass (23),
- said methanization digester (7) being surmounted by a grasping means (20) which can permit the supply of the solid biomass (23) and discharge of the residual digested solid biomass (19),
- said digester being connected, by means of a liquid digestate (5) supply circuit (6b) and by means of a circuit (14) for discharge of biogas (25) and liquid digestate (5), to said main digestion unit (1).

2. The system as claimed in claim 1, **characterized in that** the grasping means (20) is a grab (21) comprising a plurality of hooks, or a grabber comprising buckets.

3. The system as claimed in claim 1 or 2, **characterized in that** the upper perforated separation element (13, 13a, 13b, 13c, 13d) is a perforated pipe, the end of which is closed partially or completely.

4. The system as claimed in any one of the preceding claims, **characterized in that** said digester (7) comprises at least one lower perforated separation element (18), situated in the lower part of the tank (8), which can separate the liquid digestate (5) from the solid biomass (23).

5. The system as claimed in claim 4, **characterized in that** the lower perforated separation element (18) is a perforated floor.

6. The system as claimed in any one of the preceding claims, **characterized in that** said digester (7) comprises an opening (16) provided with a valve (17) which can shut off or permit the passage of the air (24) between the interior and the exterior of the tank (8) of the digester (7), and a valve (15) situated on a conveying circuit (14), which can shut off or permit the circulation of biogas (25) and liquid digestate (5), between the digester (7) and a main digestion unit (1).

7. The system as claimed in any one of the preceding claims, **characterized in that** said digester (7) comprises a liquid stopper (26) positioned on the roof (10) of the tank (8).

8. The system as claimed in any one of the preceding claims, **characterized in that** the main digestion unit (1) is constituted by two distinct elements, one designed to contain mainly liquid digestate (5), and the other designed to contain mainly biogas (25).

9. The system as claimed in any one of the preceding claims, **characterized in that** the discharge circuit (14) comprises a circuit (14a) which can convey biogas (25), and a circuit (14b) which can convey liquid digestate (25).

10. A process for production of biogas (25) comprising at least the following successive steps:
- a step of supplying solid biomass (23), implemented by at least one grasping means (20), through an area (9) for intake and discharge situated in the upper part of a tank (8) of a digester (7), as defined in claim 1, and on which a roof (10) can open and close;
- a step of filling the tank (8) of the digester (7) with a liquid digestate (5), in order to immerse the solid biomass (23);
- a step of digestion constituted by percolation of the liquid digestate (5) through the solid biomass (23), and diffusion of the liquid digestate (5) in the solid biomass (23) in anaerobic conditions, in order to generate the biogas (25), then recuperate it through a discharge area (9') situated in the upper part of said digester (7); and
- a step of aerobic emptying of the liquid digestate (5) from the tank (8) of the digester (7); and
- a step of discharge of the residual digested solid biomass (19), implemented by at least one grasping means (20), through the area (9) of the digester (7).

11. The process for production of biogas (25) as claimed in claim 10, **characterized in that** it additionally comprises at least the following steps between the step of digestion and the step of aerobic emptying of the liquid digestate (5):
- a step of anaerobic emptying of the liquid digestate (5) from the tank (8) of the digester (7); and
- a step of refilling of the tank (8) of the digester (7) with liquid digestate (5) after anaerobic emptying.

12. The process for production of biogas (25) as claimed in claim 11, **characterized in that** it additionally comprises, after the step of re-filling of the tank (8) of said digester (7) with a liquid digestate (5), at least one step of digestion constituted by percolation of the liquid digestate (5) through the solid biomass (23), and diffusion of the liquid digestate (5) in the solid biomass (23), in order to generate then recuperate the biogas (25) through the area (9').
